# EUROPEAN PATENT APPLICATION

(11) **EP 4 403 553 A1**
(43) Date of publication of application: **24.07.2024**
(21) Application number: 22869232.3
(22) Date of filing: 14.09.2022
(51) Int. Cl.: C07D 401/14, C07D 401/04, A61K 31/506, A61K 31/444, A61P 35/00, A61P 37/00

(54) **POLYMORPH AND APPLICATION OF PYRIMIDINE DERIVATIVE AND PHARMACEUTICALLY ACCEPTABLE SALT THEREOF**

(30) Priority: 15.09.2021 CN 202111079644
(71) Applicant: Shanghai Haiyan Pharmaceutical Technology Co., Ltd., Shanghai 201203 (CN); Yangtze River Pharmaceutical Group Co., Ltd., Taizhou, Jiangsu 225321 (CN)
(72) Inventor: ZHAO, Shuangni, Shanghai 201203 (CN); JIANG, Taotao, Shanghai 201203 (CN); WANG, Jibiao, Shanghai 201203 (CN); TAO, Tao, Shanghai 201203 (CN)
(74) Representative: Ostertag & Partner Patentanwälte mbB
(86) International application number: PCT/CN2022/118594
(87) International publication number: WO 2023/040863

(57) **Abstract**

Provided is a polymorph and application of a pyrimidine derivative and a pharmaceutically acceptable salt thereof. The polymorph and application of the pyrimidine derivative that is an A_{2A}/A_{2B} dual receptor antagonist having the chemical name 3-(4-amino-5-fluoro-6-(1-((6-(2-hydroxypropan-2-yl)pyridin-2-yl)methyl)-1H-1,2,3-triazol-4-yl)pyrimidine-2-yl)-2-methylbenzonitrile and the pharmaceutically acceptable salt thereof.

## Description

### RELATED APPLICATION

This application claims priority of Chinese patent application No. CN202111079644.0, filed on September 15, 2021, titled "POLYMORPH AND APPLICATION OF PYRIMIDINE DERIVATIVE AND PHARMACEUTICALLY ACCEPTABLE SALT THEREOF", which is hereby incorporated by reference in its entirety.

### TECHNICAL FIELD

The present application relates to the field of pharmaceutical technology, in particular to a polymorph of a pyrimidine derivative and a pharmaceutically acceptable salt thereof, and use thereof. Among other things, the present application relates to a polymorph of 3-(4-amino-5-fluoro-6-(1-((6-(2-hydroxypropan-2-yl)pyridin-2-yl)methyl)-1H-1,2,3-triazol-4-yl)pyrimidin-2-yl)-2-methylbenzonitrile and a pharmaceutically acceptable salt thereof, and use thereof.

### BACKGROUND

Adenosine is an endogenous nucleoside found throughout human cells, consisting of adenine and ribose, which is widely distributed both intracellularly and extracellularly. Adenosine is involved in a variety of physiological and biochemical functions in the body. For example, adenosine can enter the myocardium directly and is phosphorylated to produce adenosine triphosphate (ATP), which is involved in energy metabolism in the myocardium. In the Central Nervous System (CNS), adenosine can control the release of neurotransmitters and post-synaptic neuronal responses, regulate movement, protect neurons, and influence important life processes such as sleep and wakefulness. In pathological states, extracellular adenosine concentration increases significantly under conditions of tumor or hypoxia. Adenosine may play an important role in tumor immunosuppression by promoting tumor angiogenesis, proliferation, development, and tumor migration.

Adenosine Receptor (AR) belongs to the family of Guanosine-binding Protein Coupled Receptor (GPCR), the endogenous ligand of which is adenosine. The currently known adenosine receptors consist of four subtypes of receptors, A1, A2_{A}, A2_{B} and A3. Binding of adenosine to A1 or A3 receptors can inhibit the production of cyclic adenosine monophosphate (cAMP), while binding of adenosine to A2_{A} or A2_{B} receptors can activate adenosine activating enzyme, which in turn can up-regulate the level of cAMP, and play further physiological regulatory roles.

Both A1 and A3 receptors are mainly expressed in the central nervous system, while the A2_{A} and A2_{B} adenosine receptors are expressed in both the central nervous system and the peripheral system. In the tumor microenvironment, the A2_{A} and A2_{B} adenosine receptors are widely expressed in immune cells and have strong immunosuppressive functions. Therefore, it is extremely valuable to develop a medicament around these two targets for the prevention or treatment of tumors or immune-related diseases.

In order to better meet the market demand, a compound with A2_{A}/A2_{B} receptor antagonistic activity, a pharmaceutically acceptable salt thereof and a polymorph thereof are further developed to facilitate further medicinal development.

### SUMMARY

The objects of the present application include providing a polymorph of an A2_{A}/A_{2B} receptor antagonist or a pharmaceutically acceptable salt thereof, and use thereof.

In a first aspect of the present application, there is provided a polymorph of a compound of formula X or a pharmaceutically acceptable salt of the compound of formula X, wherein the compound of formula X is a pyrimidine derivative having the structure shown in formula X as follows,

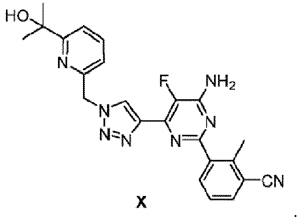

In the first aspect of the present application, both the polymorph of the compound of formula X and polymorph of the pharmaceutically acceptable salt of the compound of formula X are included.

The pharmaceutically acceptable salt is a salt formed from the compound of formula X and an acid, and thus includes ingredients of the compound of formula X and the acid.

In some embodiments, the pharmaceutically acceptable salt is an inorganic salt. Accordingly, the acid used for salt formation is an inorganic acid.

In some embodiments, the pharmaceutically acceptable salt is selected from hydrochloride, sulfate, and hydrobromide. Accordingly, the acid used for salt formation is selected from hydrochloric acid, sulfuric acid and hydrobromic acid.

In some embodiments, the polymorph of the compound of formula X and the polymorph of the pharmaceutically acceptable salt of the compound of formula X are each independently in an anhydrous form, a hydrate form, or a solvate form.

In some embodiments, X-ray powder diffraction patterns are obtained using Cu-Kα radiation.

Each of the X-ray powder diffraction patterns involved in the present application can be independently obtained using Cu-Kα radiation.

In some embodiments, the polymorph is a type I crystal of the compound of formula X, i.e., free alkali crystal form I, the X-ray powder diffraction pattern of which has peaks at diffraction angle 2θ (°) of the group comprising: 18.08 ± 0.2, 21.41 ± 0.2, and 24.83 ± 0.2.

In some embodiments, the X-ray powder diffraction pattern of the free alkali crystal form I further comprises at least 1 peak at diffraction angle 2θ (°) selected from: 12.90±0.2, 15.26±0.2, 16.47±0.2, 17.81±0.2, and 19.57±0.2.

In some embodiments, the X-ray powder diffraction pattern of the free alkali crystal form I further comprises at least 1 peak at diffraction angle 2θ (°) selected from: 22.01 ± 0.2 and 25.43 ± 0.2.

In some embodiments, the X-ray powder diffraction pattern of the free alkali crystal form I has peaks at diffraction angle 2θ (°) of the group comprising: 18.08±0.2, 21.41±0.2 and 24.83±0.2, and further comprises 2 or more peaks at diffraction angle 2θ (°) selected from: 12.90±0.2, 15.26±0.2, 16.47±0.2, 17.81±0.2, 19.57±0.2, 22.01±0.2 and 25.43±0.2.

In some embodiments, the X-ray powder diffraction pattern of the free alkali crystal form I further comprises 2 or more peaks at diffraction angle 2θ (°) selected from: 9.50±0.2, 10.13±0.2, 12.53±0.2, 18.89±0.2, 19.94±0.2 and 20.33±0.2.

In some embodiments, the X-ray powder diffraction pattern of the free alkali crystal form I further comprises 2 or more peaks at diffraction angle 2θ (°) selected from: 7.73±0.2, 13.94±0.2 and 16.94±0.2.

In some embodiments, the X-ray powder diffraction pattern of the free alkali crystal form I further comprises 2 or more peaks at diffraction angle 2θ (°) selected from: 27.84±0.2, 28.49±0.2, 29.27±0.2, 30.38±0.2, 30.86±0.2 and 32.03±0.2.

In some embodiments, the X-ray powder diffraction pattern of the free alkali crystal form I has diffraction peaks at 2θ (°) as shown in Table 1, and the relative intensity of each diffraction peak is shown in Table 1.

**Table 1. Positions and relative intensities of major diffraction peaks in one X-ray powder diffraction pattern of free alkali crystal form I.**

| 2θ(°) | I/I₀ | 2θ(°) | I/I₀ | 2θ(°) | I/I₀ |
|---|---|---|---|---|---|
| 7.73 | W | 17.81 | S | 25.43 | S |
| 9.50 | M | 18.08 | VS | 27.84 | M |
| 10.13 | M | 18.89 | M | 28.49 | M |
| 12.53 | M | 19.57 | S | 29.27 | M |
| 12.90 | S | 19.94 | M | 30.38 | M |
| 13.94 | W | 20.33 | M | 30.86 | M |
| 15.26 | S | 21.41 | VS | 32.03 | M |
| 16.47 | S | 22.01 | S | | |
| 16.94 | W | 24.83 | VS | | |

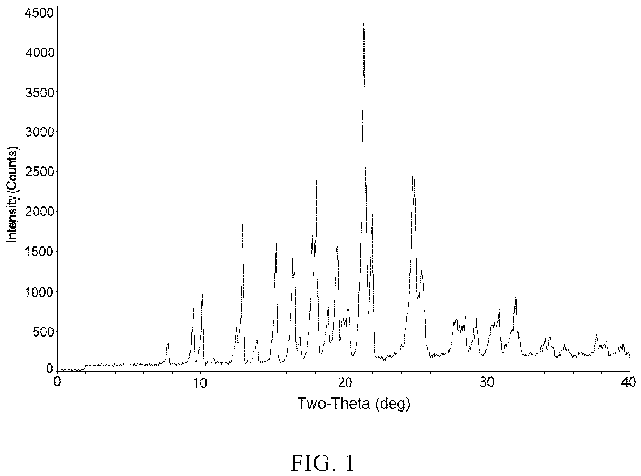

In some embodiments, the X-ray powder diffraction pattern of the free alkali crystal form I is substantially as characterized in FIG. 1.

In one embodiment, the X-ray powder diffraction pattern of the free alkali crystal form I is as characterized in FIG. 1.

In some embodiments, the free alkali crystal form I is anhydrous.

In some embodiments, the differential scanning calorimetry curve of the free alkali crystal form I has an endothermic peak at 190.15°C ± 3°C corresponding to a fusion heat of about 100.48 J/g.

In some embodiments, the differential scanning calorimetry curve of the free alkali crystal form I has an endothermic peak at 190.15°C ± 1°C.

In some embodiments, the differential scanning calorimetry curve of the free alkali crystal form I has an endothermic peak at 190.15°C ± 0.5°C.

In some embodiments, the TGA plot of the free alkali crystal form I shows almost no mass loss until 150°C and a mass loss of 0.123% at from 150°C to 210°C.

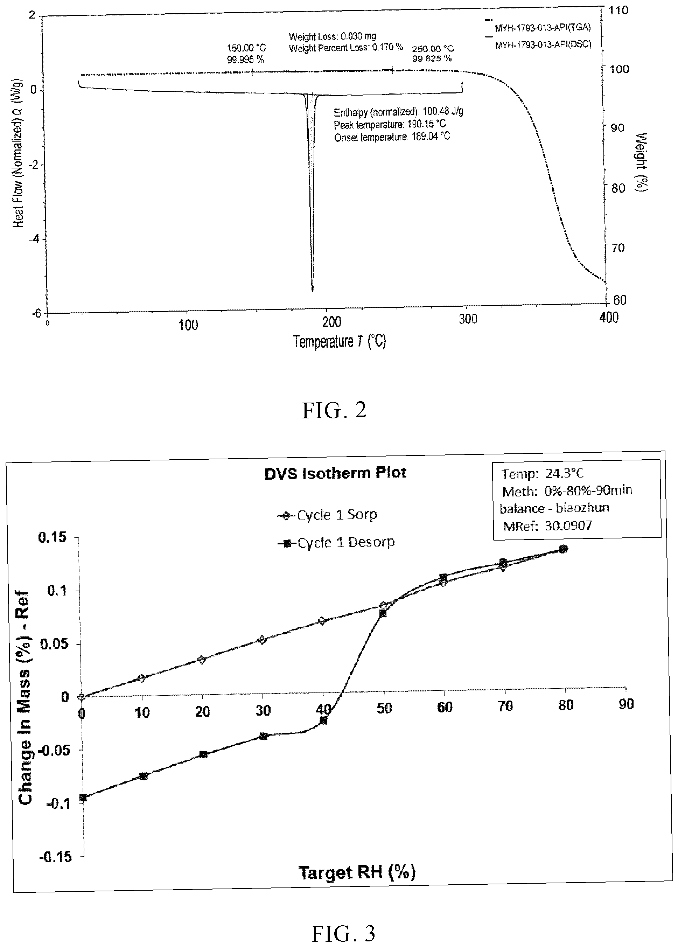

In some embodiments, the free alkali crystal form I has a TGA-DSC plot substantially as shown in FIG. 2.

In one embodiment, the free alkali crystal form I has a TGA-DSC plot as shown in FIG. 2.

In some embodiments, the free alkali crystal form I has a change in mass of less than 0.2% due to absorption during a change in relative humidity from 0% to 80% RH at 25°C, and has almost no hygroscopicity, wherein the percentage "%" of change in mass is a mass percentage.

In some embodiments, the free alkali crystal form I has a Dynamic Vapor Sorption plot (DVS plot) substantially as shown in FIG. 3.

In one embodiment, the free alkali crystal form I has a DVS plot as shown in FIG. 3.

In some embodiments, the infrared spectrum of the free alkali crystal form I has absorption peaks at about 3490 cm⁻¹, about 3280 cm⁻¹, about 3132 cm⁻¹, about 2972 cm⁻¹, about 2931 cm⁻¹, about 2210 cm⁻¹, about 1636 cm⁻¹ to 1451 cm⁻¹, about 1399 cm⁻¹, about 1366 cm⁻¹, about 855 cm⁻¹, about 799 cm⁻¹ and about 769 cm⁻¹. Herein, "about" can each independently be ±5 cm⁻¹, ±3 cm⁻¹ or ±1 cm⁻¹.

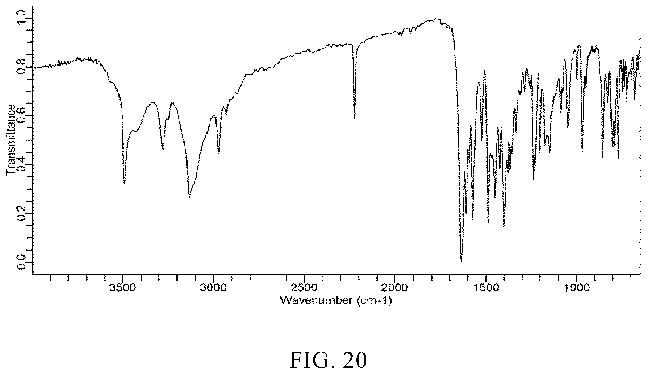

In some embodiments, the infrared spectrum of the free alkali crystal form I is substantially as characterized in FIG. 20.

In some embodiments, the free alkali crystal form I has an infrared spectrum as characterized in FIG. 20.

In some embodiments, the free alkali crystal form I has one or more features selected from:
(1) having a TGA-DSC plot substantially as characterized in FIG. 2;
(2) having a DVS plot substantially as characterized in FIG. 3; and
(3) having an infrared spectrum substantially as characterized in FIG. 20.

In some embodiments, the polymorph is a type IV crystal of the compound of formula X, i.e., free alkali crystal form IV, the X-ray powder diffraction pattern of which has peaks at diffraction angle 2θ (°) of the group comprising: 13.04 ± 0.2, 15.80 ± 0.2, 16.46 ± 0.2, and 23.89 ± 0.2.

In some embodiments, the X-ray powder diffraction pattern of the free alkali crystal form IV further comprises at least 1 peak at diffraction angle 2θ (°) selected from: 6.32 ± 0.2, 9.08 ± 0.2, 9.58 ± 0.2, and 14.12 ± 0.2.

In some embodiments, the X-ray powder diffraction pattern of the free alkali crystal form IV further comprises at least 1 peak at diffraction angle 2θ (°) selected from: 20.14 ± 0.2, 20.59 ± 0.2, 23.89 ± 0.2, and 27.53 ± 0.2.

In some embodiments, the X-ray powder diffraction pattern of the free alkali crystal form IV has peaks at diffraction angle 2θ (°) of the group comprising: 13.04±0.2, 15.80±0.2, 16.46±0.2, and 23.89±0.2, and further comprises two or more peaks at diffraction angle 2θ (°) selected from: 6.32±0.2, 9.08±0.2, 9.58±0.2, 14.12±0.2, 20.14±0.2, 20.59±0.2, 23.89±0.2 and 27.53±0.2.

In some embodiments, the X-ray powder diffraction pattern of the free alkali crystal form IV has peaks at diffraction angle 2θ (°) of the group comprising: 13.04±0.2, 15.80±0.2, 16.46±0.2, and 23.89±0.2, and further comprises two or more peaks at diffraction angle 2θ (°) selected from: 6.32±0.2, 9.08±0.2, 9.58±0.2, 14.12±0.2, 20.14±0.2, 20.59±0.2, 23.89±0.2, 27.53±0.2 and 37.76±0.2.

In some embodiments, the X-ray powder diffraction pattern of the free alkali crystal form IV further comprises 1 or 2 peaks at diffraction angle 2θ (°) selected from: 7.64 ± 0.2 and 8.34 ± 0.2.

In some embodiments, the X-ray powder diffraction pattern of the free alkali crystal form IV has peaks at 2θ (°) as shown in Table 2, and the relative intensity of each peak is as shown in Table 2.

**Table 2. Positions and relative intensities of major diffraction peaks in one X-ray powder diffraction pattern of free alkali crystal form IV.**

| 2θ(°) | I/I₀ | 2θ(°) | I/I₀ | 2θ(°) | I/I₀ |
|---|---|---|---|---|---|
| 6.32 | S | 13.04 | VS | 20.59 | S |
| 7.64 | M | 14.12 | S | 23.89 | VS |
| 8.34 | M | 15.80 | VS | 27.53 | S |
| 9.08 | S | 16.46 | VS | 37.76 | S |
| 9.58 | S | 20.14 | S | | |

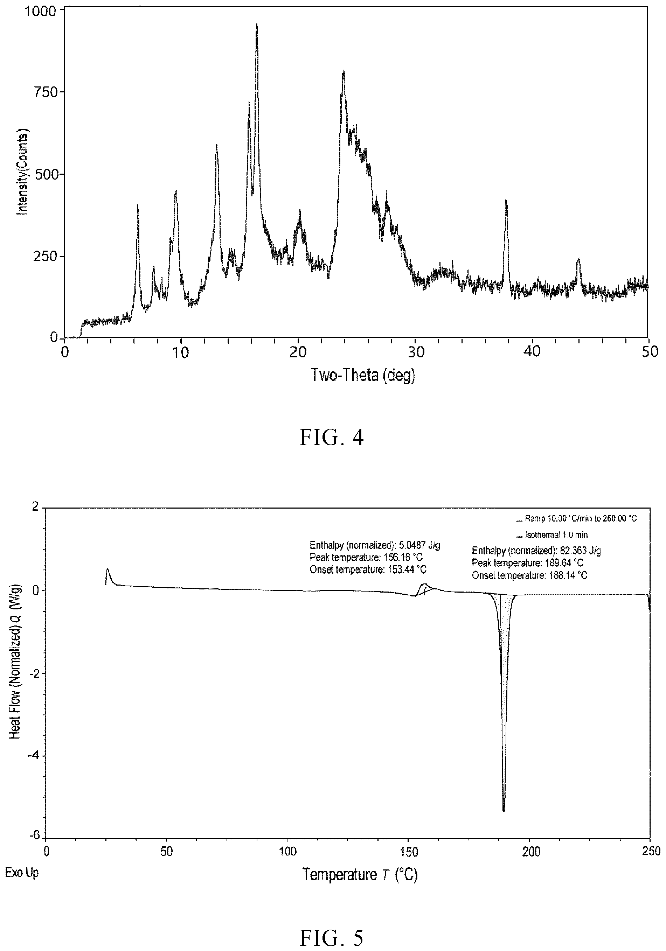

In some embodiments, the X-ray powder diffraction pattern of the free alkali crystal form IV is substantially as characterized in FIG. 4.

In one embodiment, the X-ray powder diffraction pattern of the free alkali crystal form IV is as characterized in FIG. 4.

In some embodiments, the differential scanning calorimetry curve of the free alkali crystal form IV has an endothermic peak at 189.64°C ± 3°C.

In some embodiments, the differential scanning calorimetry curve of the free alkali crystal form IV has an endothermic peak at 189.64°C ± 1°C.

In some embodiments, the differential scanning calorimetry curve of the free alkali crystal form IV has an endothermic peak at 189.64°C ± 0.5°C.

In some embodiments, the free alkali crystal form IV has a differential scanning calorimetry curve substantially as shown in FIG. 5.

In one embodiment, the free alkali crystal form IV has a differential scanning calorimetry curve as shown in FIG. 5.

In some embodiments, the polymorph is a type V crystal of the compound of formula X, i.e., free alkali crystal form V, the X-ray powder diffraction pattern of which has peaks at diffraction angle 2θ (°) of the group comprising: 6.17±0.2, 9.37±0.2, 10.39±0.2, 11.65±0.2, 14.35±0.2, 15.74±0.2, and 17.21±0.2.

In some embodiments, the X-ray powder diffraction pattern of the free alkali crystal form V further comprises 2 or more peaks at diffraction angle 2θ (°) selected from: 21.65±0.2, 22.31±0.2, 24.55±0.2, 24.86±0.2, 25.70±0.2, 26.08±0.2, and 27.31±0.2.

In some embodiments, the X-ray powder diffraction pattern of the free alkali crystal form V further has a peak at diffraction angle 2θ (°) of: 38.71 ± 0.2.

In some embodiments, the X-ray powder diffraction pattern of the free alkali crystal form V comprises 1 or 2 peaks at diffraction angle 2θ (°) selected from: 38.71 ± 0.2 and 43.60 ± 0.2.

In some embodiments, the X-ray powder diffraction pattern of the free alkali crystal form V further comprises 2 or more peaks at diffraction angle 2θ (°) selected from: 6.91 ± 0.2, 8.08 ± 0.2, 8.70 ± 0.2, 12.77 ±0.2, and 13.25 ± 0.2.

In some embodiments, the X-ray powder diffraction pattern of the free alkali crystal form V further comprises 2 or more peaks at diffraction angle 2θ (°) selected from: 19.01 ± 0.2, 19.43 ± 0.2, 21.35 ± 0.2, 23.15 ± 0.2, 29.38 ± 0.2, 32.03 ± 0.2, 32.29 ± 0.2, 35.08 ± 0.2, 39.80±0.2 and 40.19±0.2.

In some embodiments, the X-ray powder diffraction pattern of the free alkali crystal form V comprises 2 or more peaks at diffraction angle 2θ (°) selected from: 19.01 ± 0.2, 19.43 ± 0.2, 21.35 ± 0.2, 23.15 ± 0.2, 29.38 ± 0.2, 32.03 ± 0.2, 32.29 ± 0.2, 35.08 ± 0.2, 37.43±0.2, 39.80±0.2 and 40.19±0.2.

In some embodiments, the X-ray powder diffraction pattern of the free alkali crystal form V has peaks at 2θ (°) as shown in Table 3, and the relative intensity of each peak is as shown in Table 3.

**Table 3. Positions and relative intensities of major diffraction peaks in one X-ray powder diffraction pattern of free alkali crystal form V.**

| 2θ(°) | I/I₀ | 2θ(°) | I/I₀ | 2θ(°) | I/I₀ |
|---|---|---|---|---|---|
| 6.17 | S | 17.21 | S | 27.31 | S |
| 6.91 | M | 19.01 | M | 29.38 | M |
| 8.08 | M | 19.43 | M | 32.03 | M |
| 8.70 | M | 21.35 | M | 32.29 | M |
| 9.37 | S | 21.65 | S | 35.08 | M |
| 10.39 | S | 22.31 | S | 37.43 | M |
| 11.65 | S | 23.15 | M | 38.71 | S |
| 12.77 | M | 24.55 | S | 39.80 | M |
| 13.25 | M | 24.86 | S | 40.19 | M |
| 14.35 | VS | 25.70 | S | 43.60 | S |
| 15.74 | S | 26.08 | S | | |

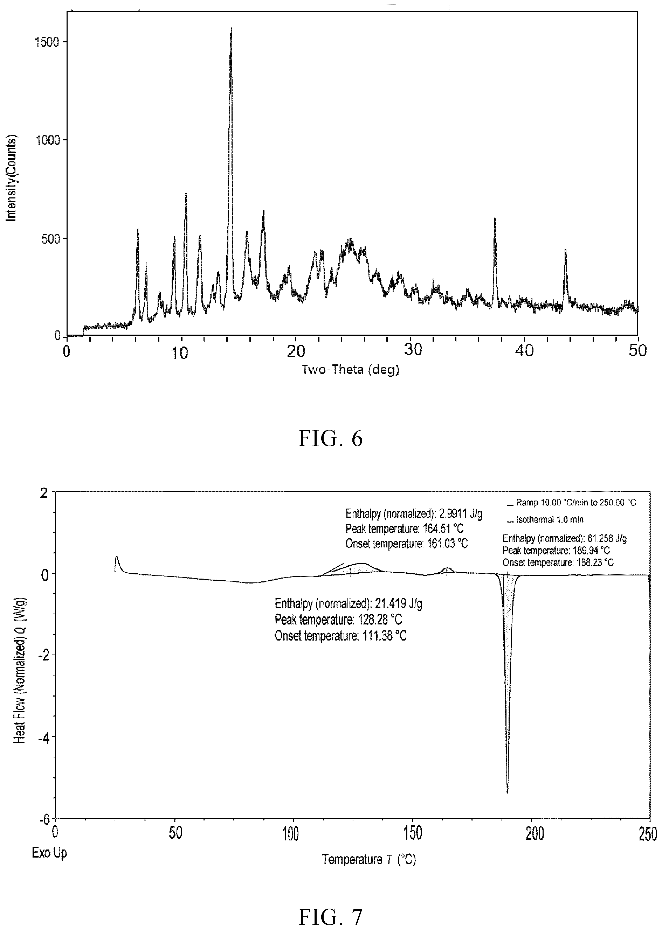

In some embodiments, the X-ray powder diffraction pattern of the free alkali crystal form V is substantially as characterized in FIG. 6.

In one embodiment, the X-ray powder diffraction pattern of the free alkali crystal form V is as characterized in FIG. 6.

In some embodiments, the differential scanning calorimetry curve of the free alkali crystal form V has an endothermic peak at 189.94°C ± 3°C.

In some embodiments, the differential scanning calorimetry curve of the free alkali crystal form V has an endothermic peak at 189.94°C ± 1°C.

In some embodiments, the differential scanning calorimetry curve of the free alkali crystal form V has an endothermic peak at 189.94°C ± 0.5°C.

In some embodiments, the free alkali crystal form V has a differential scanning calorimetry curve substantially as shown in FIG. 7.

In one embodiment, the free alkali crystal form V has a differential scanning calorimetry curve as shown in FIG. 7.

In some embodiments, the polymorph is a type VI crystal of the compound of formula X, i.e., free alkali crystal form VI, the X-ray powder diffraction pattern of which has peaks at diffraction angle 2θ (°) of the group comprising: 12.55 ± 0.2, 14.86 ± 0.2, 16.15 ± 0.2, 17.69 ± 0.2, 21.08 ± 0.2, 21.58 ± 0.2, 24.53 ± 0.2 and 25.01±0.2.

In some embodiments, the X-ray powder diffraction pattern of the free alkali crystal form VI further comprises 2 or more peaks at diffraction angle 2θ (°) selected from: 9.04±0.2, 9.68±0.2, 13.37±0.2, 18.53±0.2, 19.19±0.2, 19.64±0.2, and 19.97±0.2.

In some embodiments, the X-ray powder diffraction pattern of the free alkali crystal form VI further comprises 2 or more peaks at diffraction angle 2θ (°) selected from: 23.69±0.2, 27.55±0.2, 30.34±0.2, and 31.46±0.2.

In some embodiments, the X-ray powder diffraction pattern of the free alkali crystal form VI further comprises 2 or more peaks at diffraction angle 2θ (°) selected from: 9.04±0.2, 9.68±0.2, 13.37±0.2, 18.53±0.2, 19.19±0.2, 19.64±0.2, 19.97±0.2, 23.69 ± 0.2, 27.55 ± 0.2, 30.34 ± 0.2, 31.46 ± 0.2 and 37.42 ± 0.2.

In some embodiments, the X-ray powder diffraction pattern of the free alkali crystal form VI further comprises 1 or 2 peaks at diffraction angle 2θ (°) selected from: 7.24 ± 0.2 and 12.07 ± 0.2.

In some embodiments, the X-ray powder diffraction pattern of the free alkali crystal form VI has peaks at 2θ (°) as shown in Table 4, and the relative intensity of each peak is as shown in Table 4.

**Table 4. Positions and relative intensities of major diffraction peaks in one X-ray powder diffraction pattern of free alkali crystal form VI.**

| 2θ(°) | I/I₀ | 2θ(°) | I/I₀ | 2θ(°) | I/I₀ |
|---|---|---|---|---|---|
| 7.24 | M | 17.69 | VS | 24.53 | VS |
| 9.04 | S | 18.53 | S | 25.01 | VS |
| 9.68 | S | 19.19 | S | 27.55 | S |
| 12.07 | M | 19.64 | S | 30.34 | S |
| 12.55 | VS | 19.97 | S | 31.46 | S |
| 13.37 | S | 21.08 | VS | 37.42 | S |
| 14.86 | VS | 21.58 | VS | | |
| 16.15 | VS | 23.69 | S | | |

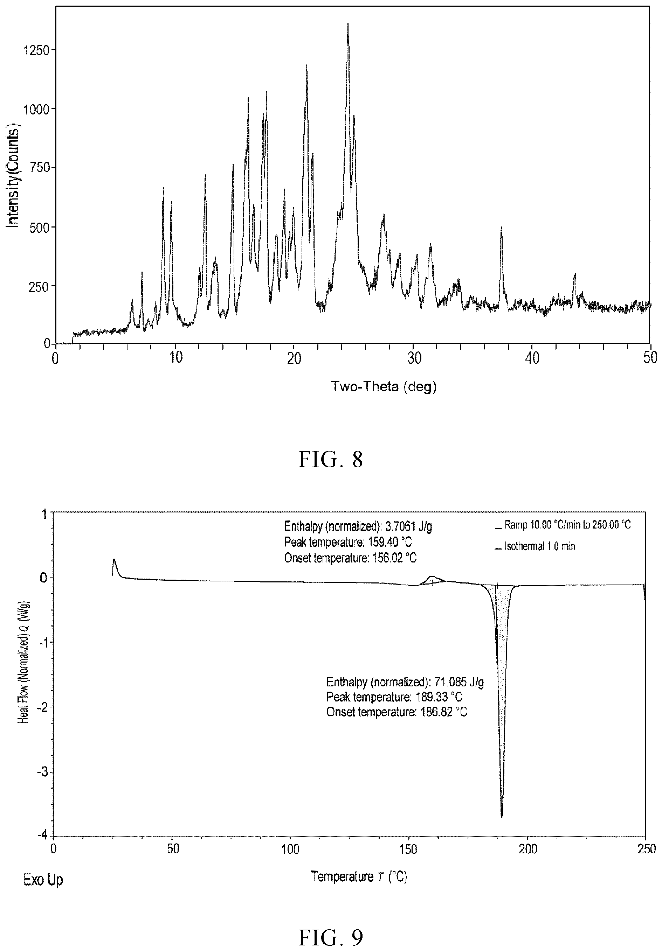

In some embodiments, the X-ray powder diffraction pattern of the free alkali crystal form VI is substantially as characterized in FIG. 8.

In one embodiment, the X-ray powder diffraction pattern of the free alkali crystal form VI is as characterized in FIG. 8.

In some embodiments, the differential scanning calorimetry curve of the free alkali crystal form VI has an endothermic peak at 189.33°C ± 3°C.

In some embodiments, the differential scanning calorimetry curve of the free alkali crystal form VI has an endothermic peak at 189.33°C ± 1°C.

In some embodiments, the differential scanning calorimetry curve of the free alkali crystal form VI has an endothermic peak at 189.33°C ± 0.5°C.

In some embodiments, the free alkali crystal form VI has a differential scanning calorimetry curve substantially as shown in FIG. 9.

In one embodiment, the free alkali crystal form VI has a differential scanning calorimetry curve as shown in FIG. 9.

In some embodiments, the polymorph is a type VII crystal of the compound of formula X, i.e., free alkali crystal form VII, the X-ray powder diffraction pattern of which has peaks at diffraction angle 2θ (°) of the group comprising: 14.92±0.2, 16.13±0.2, 17.59±0.2, 20.87±0.2, 21.20±0.2, 21.71±0.2, 24.12±0.2, 24.62±0.2 and 25.12±0.2.

In some embodiments, the polymorph is a type VII crystal of the compound of formula X, i.e., free alkali crystal form VII, the X-ray powder diffraction pattern of which has peaks at diffraction angle 2θ (°) of the group comprising: 14.92±0.2, 16.13±0.2, 17.59±0.2, 20.87±0.2, 21.20±0.2, 21.71±0.2, 24.12±0.2, 24.62±0.2, 25.12±0.2, 37.40±0.2 and 43.55±0.2.

In some embodiments, the X-ray powder diffraction pattern of the free alkali crystal form VII further comprises 2 or more peaks at diffraction angle 2θ (°) selected from: 6.17±0.2, 9.07±0.2, 9.67±0.2, 10.37±0.2, 12.61±0.2, 14.39±0.2, 19.21±0.2, 19.73± 0.2 and 20.05±0.2.

In some embodiments, the X-ray powder diffraction pattern of the free alkali crystal form VII further comprises 2 or more peaks at diffraction angle 2θ (°) selected from: 27.25±0.2, 27.39±0.2, 27.76±0.2, 28.97±0.2, 30.36±0.2, 31.25±0.2, and 31.67±0.2.

In some embodiments, the X-ray powder diffraction pattern of the free alkali crystal form VII further comprises 2 or more peaks at diffraction angle 2θ (°) selected from: 5.00±0.2, 6.91±0.2, 7.19±0.2, 11.58±0.2, 13.46±0.2, 33.47±0.2, and 34.98±0.2.

In some embodiments, the X-ray powder diffraction pattern of the free alkali crystal form VII has peaks at 2θ (°) as shown in Table 5, and the relative intensity of each peak is as shown in Table 5.

**Table 5. Positions and relative intensities of major diffraction peaks in one X-ray powder diffraction pattern of free alkali crystal form VII.**

| 2θ(°) | I/I₀ | 2θ(°) | I/I₀ | 2θ(°) | I/I₀ |
|---|---|---|---|---|---|
| 5.00 | M | 16.13 | VS | 27.39 | S |
| 6.17 | S | 17.59 | VS | 27.76 | S |
| 6.91 | M | 19.21 | S | 28.97 | S |
| 7.19 | M | 19.73 | S | 30.36 | S |
| 9.07 | S | 20.05 | S | 31.25 | S |
| 9.67 | S | 20.87 | VS | 31.67 | S |
| 10.37 | S | 21.20 | VS | 33.47 | M |
| 11.58 | M | 21.71 | VS | 34.98 | M |
| 12.61 | S | 24.12 | VS | 37.40 | VS |
| 13.46 | M | 24.62 | VS | 43.55 | VS |
| 14.39 | S | 25.12 | VS | | |
| 14.92 | VS | 27.25 | S | | |

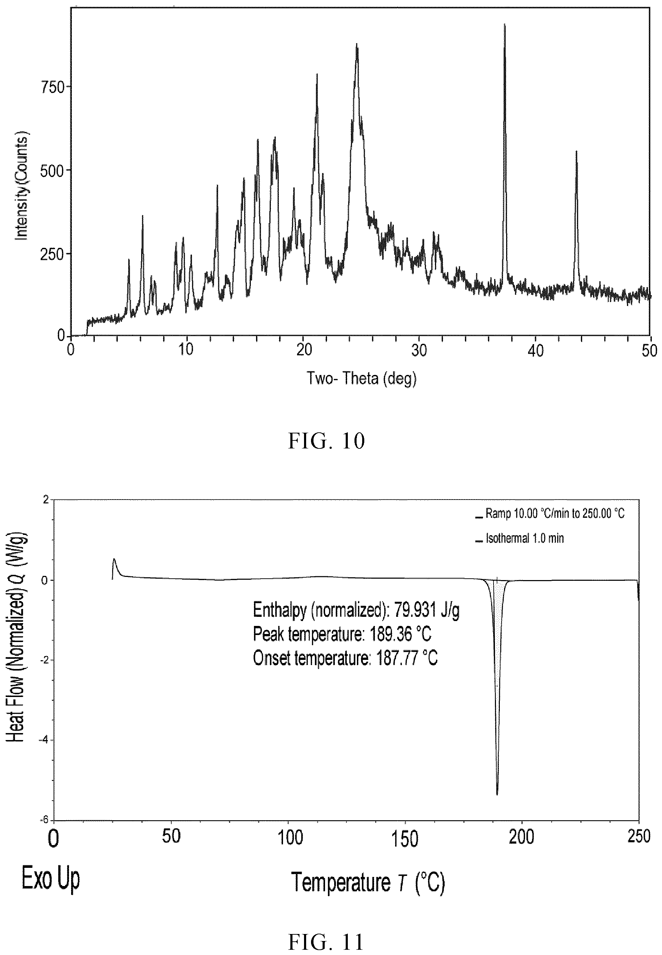

In some embodiments, the X-ray powder diffraction pattern of the free alkali crystal form VII is substantially as characterized in FIG. 10.

In one embodiment, the X-ray powder diffraction pattern of the free alkali crystal form VII is as characterized in FIG. 10.

In some embodiments, the differential scanning calorimetry curve of the free alkali crystal form VII has an endothermic peak at 189.36°C ± 3°C.

In some embodiments, the differential scanning calorimetry curve of the free alkali crystal form VII has an endothermic peak at 189.36°C ± 1°C.

In some embodiments, the differential scanning calorimetry curve of the free alkali crystal form VII has an endothermic peak at 189.36°C ± 0.5°C.

In some embodiments, the free alkali crystal form VII has a differential scanning calorimetry curve substantially as shown in FIG. 11.

In one embodiment, the free alkali crystal form VII has a differential scanning calorimetry curve as shown in FIG. 11.

In some embodiments, the free alkali polymorph is a type I crystal of hydrochloride of the compound of formula X, i.e., hydrochloride crystal form I, the X-ray powder diffraction pattern of which has peaks at diffraction angle 2θ (°) of the group comprising: 13.12 ± 0.2, 13.91 ± 0.2, 17.62 ± 0.2, 22.58 ± 0.2, and 26.51 ± 0.2.

In some embodiments, the X-ray powder diffraction pattern of the hydrochloride crystal form I further comprises 2 or more peaks at diffraction angle 2θ (°) selected from: 8.39±0.2, 10.18±0.2, 15.25±0.2, 18.64±0.2, 20.96±0.2, 25.52±0.2, 27.01±0.2 and 29.48± 0.2.

In some embodiments, the X-ray powder diffraction pattern of the hydrochloride crystal form I further comprises 2 or more peaks at diffraction angle 2θ (°) selected from: 11.44±0.2, 12.63±0.2, 17.27±0.2, 18.97±0.2, 20.12±0.2, 21.61±0.2, 23.29±0.2 and 29.15± 0.2.

In some embodiments, the X-ray powder diffraction pattern of the hydrochloride crystal form I has peaks at 2θ (°) as shown in Table 6, and the relative intensity of each peak is as shown in Table 6.

**Table 6. Positions and relative intensities of major diffraction peaks in one X-ray powder diffraction pattern of hydrochloride crystal form I.**

| 2θ(°) | I/I₀ | 2θ(°) | I/I₀ | 2θ(°) | I/I₀ |
|---|---|---|---|---|---|
| 8.39 | M | 17.27 | M | 22.58 | VS |
| 10.18 | M | 17.62 | S | 23.29 | M |
| 11.44 | M | 18.64 | M | 25.52 | M |
| 12.63 | M | 18.97 | M | 26.51 | S |
| 13.12 | S | 20.12 | M | 27.01 | M |
| 13.91 | S | 20.96 | M | 29.15 | M |
| 15.25 | M | 21.61 | M | 29.48 | M |

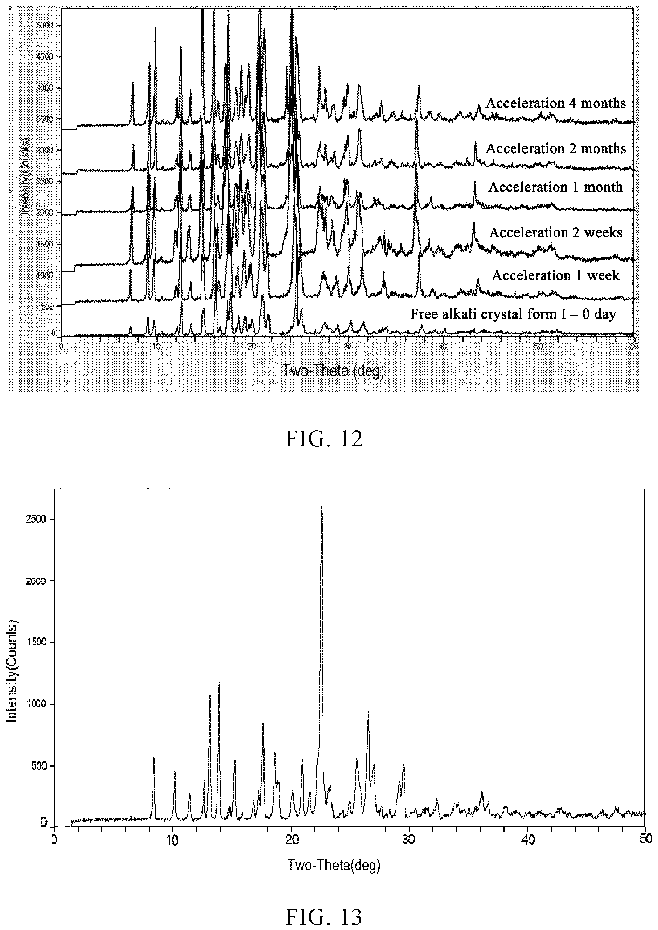

In some embodiments, the X-ray powder diffraction pattern of the hydrochloride crystal form I is substantially as characterized in FIG. 13.

In one embodiment, the X-ray powder diffraction pattern of the hydrochloride crystal form I is as characterized in FIG. 13.

In some embodiments, the differential scanning calorimetry curve of the hydrochloride crystal form I has an endothermic peak at 225.37°C ± 3°C.

In some embodiments, the differential scanning calorimetry curve of the hydrochloride crystal form I has an endothermic peak at 225.37°C ± 1°C.

In some embodiments, the differential scanning calorimetry curve of the hydrochloride crystal form I has an endothermic peak at 225.37°C ± 0.5°C.

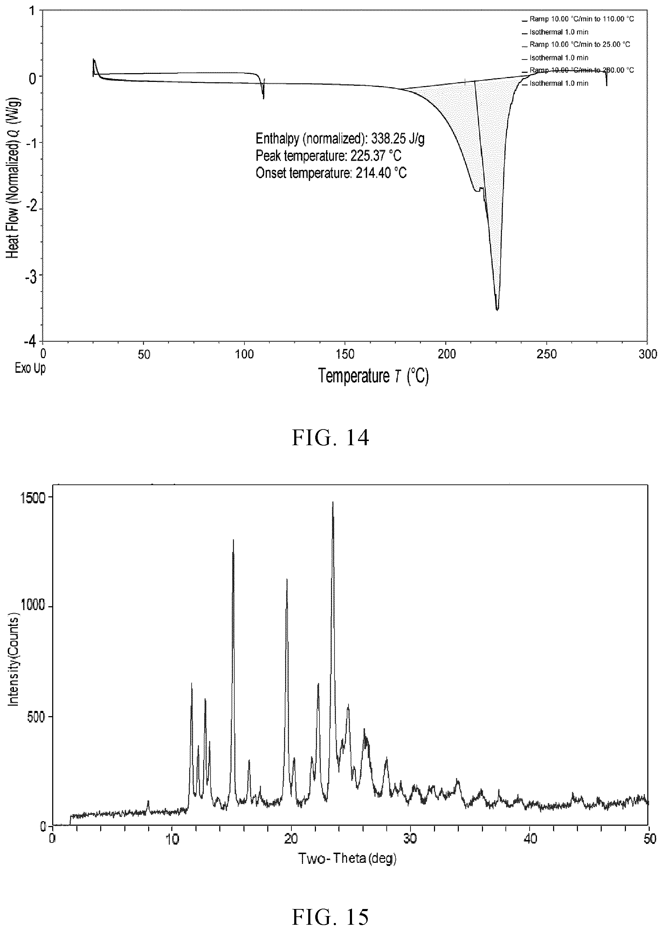

In some embodiments, the hydrochloride crystal form I has a differential scanning calorimetry curve substantially as shown in FIG. 14.

In one embodiment, the hydrochloride crystal form I has a differential scanning calorimetry curve as shown in FIG. 14.

In some embodiments, the polymorph is a type I crystal of sulfate of the compound of formula X, i.e., sulfate crystal form I, the X-ray powder diffraction pattern of which has peaks at diffraction angle 2θ (°) of the group comprising: 15.13 ± 0.2, 19.64 ± 0.2, and 23.48 ± 0.2.

In some embodiments, the X-ray powder diffraction pattern of the sulfate crystal form I further comprises 2 or more peaks at diffraction angle 2θ (°) selected from: 11.62±0.2, 12.77±0.2, 13.13±0.2, 22.25±0.2, 24.80±0.2, and 26.09±0.2.

In some embodiments, the X-ray powder diffraction pattern of the sulfate crystal form I further comprises 2 or more peaks at diffraction angle 2θ (°) selected from: 12.19 ± 0.2, 16.45 ± 0.2 and 21.71 ± 0.2.

In some embodiments, the X-ray powder diffraction pattern of the sulfate crystal form I has peaks at 2θ (°) as shown in Table 7, and the relative intensity of each peak is as shown in Table 7.

**Table 7. Positions and relative intensities of major diffraction peaks in one X-ray powder diffraction pattern of sulfate crystal form I.**

| 2θ(°) | I/I₀ | 2θ(°) | I/I₀ | 2θ(°) | I/I₀ |
|---|---|---|---|---|---|
| 11.62 | S | 15.13 | VS | 22.25 | S |
| 12.19 | M | 16.45 | M | 23.48 | VS |
| 12.77 | S | 19.64 | VS | 24.80 | S |
| 13.13 | S | 21.71 | M | 26.09 | S |

In some embodiments, the X-ray powder diffraction pattern of the sulfate crystal form I is substantially as characterized in FIG. 15.

In one embodiment, the X-ray powder diffraction pattern of the sulfate crystal form I is characterized in FIG. 15.

In some embodiments, the differential scanning calorimetry curve of the sulfate crystal form I has an endothermic peak at 205.20°C ± 3°C.

In some embodiments, the differential scanning calorimetry curve of the sulfate crystal form I has an endothermic peak at 205.20°C ± 1°C.

In some embodiments, the differential scanning calorimetry curve of the sulfate crystal form I has an endothermic peak at 205.20°C ± 0.5°C.

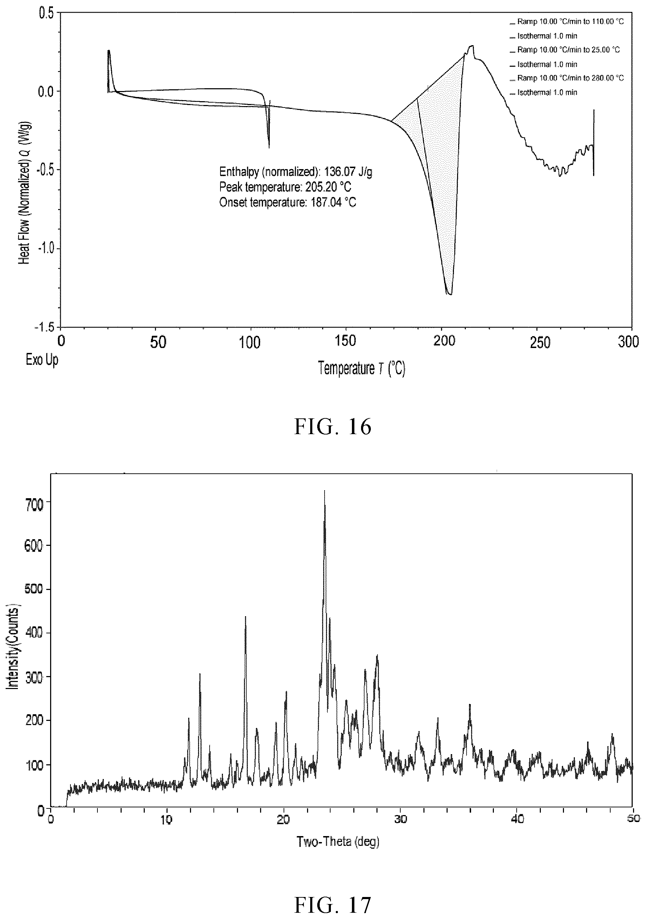

In some embodiments, the sulfate crystal form I has a differential scanning calorimetry curve substantially as shown in FIG. 16.

In one embodiment, the sulfate crystal form I has a differential scanning calorimetry curve as shown in FIG. 16.

In some embodiments, the polymorph is a type I crystal of hydrobromide of the compound of formula X, i.e., hydrobromide crystal form I, the X-ray powder diffraction pattern of which has peaks at diffraction angle 2θ (°) of the group comprising: 16.70 ± 0.2, 23.51 ± 0.2 and 23.96 ± 0.2.

In some embodiments, the X-ray powder diffraction pattern of the hydrobromide crystal form I further comprises 2 or more peaks at diffraction angle 2θ (°) selected from: 11.84±0.2, 12.79±0.2, 19.34±0.2, 20.23±0.2, 23.09±0.2, 24.34±0.2, 25.37±0.2, 26.21±0.2, 26.99±0.2, 28.04±0.2, 33.22±0.2 and 35.96±0.2.

In some embodiments, the X-ray powder diffraction pattern of the hydrobromide crystal form I further comprises 2 or more peaks at diffraction angle 2θ (°) selected from: 11.48±0.2, 13.64±0.2, 15.46±0.2, 15.96±0.2, 17.66±0.2, 18.71±0.2, 20.99±0.2, 21.51±0.2, 31.60±0.2, 31.90±0.2, 35.52±0.2, 36.98±0.2, 37.81±0.2, 39.29±0.2 and 39.73±0.2.

In some embodiments, the X-ray powder diffraction pattern of the hydrobromide crystal form I has peaks at 2θ (°) as shown in Table 8, and the relative intensity of each peak is as shown in Table 8.

**Table 8. Positions and relative intensities of major diffraction peaks in one X-ray powder diffraction pattern of hydrobromide crystal form I.**

| 2θ(°) | I/I₀ | 2θ(°) | I/I₀ | 2θ(°) | I/I₀ |
|---|---|---|---|---|---|
| 11.48 | M | 20.23 | S | 28.04 | S |
| 11.84 | S | 20.99 | M | 31.60 | M |
| 12.79 | S | 21.51 | M | 31.90 | M |
| 13.64 | M | 23.09 | S | 33.22 | S |
| 15.46 | Ivl | 23.51 | VS | 35.52 | M |
| 15.96 | M | 23.96 | VS | 35.96 | S |
| 16.70 | VS | 24.34 | S | 36.98 | M |
| 17.66 | M | 25.37 | S | 37.81 | M |
| 18.71 | M | 26.21 | S | 39.29 | M |
| 19.34 | S | 26.99 | S | 39.73 | M |

In some embodiments, the X-ray powder diffraction pattern of the hydrobromide crystal form I is substantially as characterized in FIG. 17.

In one embodiment, the X-ray powder diffraction pattern of the hydrobromide crystal form I is as characterized in FIG. 17.

In a second aspect of the present application, there is provided a preparation method for the polymorph of the compound of formula X and the pharmaceutically acceptable salt thereof, which can be used to prepare the polymorph described in the first aspect.

In the second aspect of the present application, the structure of the compound of formula X is consistent with the above structure of the compound of formula X.

In the second aspect of the present application, there is provided a preparation method for the polymorph of the compound of formula X, comprising the steps of:
(a0) dissolving the compound of formula X in a solvent to form a clarified solution; and
(b0) crystallizing the solution to prepare the polymorph of the compound of formula X.

Herein, in order to achieve "dissolving to form a clarified solution", the amount of the corresponding solvent should be controlled so as to completely dissolve the compound of formula X. If the amount is too low, a clarified solution cannot be obtained, and if the amount is too high, crystals may not be precipitated during crystallization.

In some embodiments, the solvent in the step (a0) is selected from acetonitrile, water, methanol, ethanol, isopropanol, acetone, ethyl acetate, 50% (v/v) ethanol/water solvent mixture, dimethylsulfoxide, N,N-dimethylacetamide, tetrahydrofuran, and a mixture thereof.

In some embodiments, the crystallization in the step (b0) may be selected from cooling crystallization, volatilizing crystallization and anti-solvent crystallization.

Herein, the key step in the cooling crystallization is to decrease the temperature to precipitate crystals.

Herein, the key step in the volatilizing crystallization is to volatilize the solvent to precipitate crystals.

Herein, the key step in the anti-solvent crystallization is "adding anti-solvent", i.e., the addition of an anti-solvent, a poor solvent, or a combination of an anti-solvent and a poor solvent, to precipitate crystals. The addition of an anti-solvent is preferred.

In some embodiments, the crystallization in the step (b0) is performed by slow cooling, slow volatilization or anti-solvent addition.

Herein, examples of slow cooling are such as natural cooling down to room temperature.

Herein, examples of slow volatilization are such as natural volatilization at room temperature.

In some embodiments, the prepared polymorph is the free alkali crystal form I, wherein the crystallizing is performed by cooling crystallization, volatilizing crystallization or anti-solvent crystallization.

In some embodiments, there is provided a preparation method for the free alkali crystal form I comprising the steps of:
(a1) dissolving the compound of formula X in the presence of a solvent to form a clarified solution; and
(b 1) crystallizing the solution.

In some embodiments, in step (a1), the solvent is selected from acetonitrile, water, methanol, ethanol, isopropanol, acetone, ethyl acetate, 50% ethanol (i.e., 50% (v/v) ethanol/water solvent mixture), dimethylsulfoxide, N,N-dimethylacetamide, tetrahydrofuran, and a mixture thereof.

In some embodiments, in step (a1), the solvent is acetonitrile, isopropanol, ethanol, ethyl acetate, 50% ethanol, dimethylsulfoxide, or N,N-dimethylacetamide.

In some embodiments, the compound of formula X is dissolved to form a clarified solution at temperature T1 in the step (a1). In some preferred embodiments, the temperature T1 is preferably above room temperature. For example, the compound of formula X is added to a solvent, heated to temperature T1 and then held at the same temperature for a period of time to clarify the mixed system (to achieve dissolving the compound of formula X to form a clarified solution), and the resulting clarified system is the solution. The temperature T1 can be a constant temperature condition (maintained at a constant temperature) or a temperature range (maintained at a constant temperature range). In some embodiments, the "step of dissolving the compound of formula X to obtain a clarified solution" is carried out at a temperature T1 selected from any of the following temperature ranges: 45°C to 120°C, 50°C to 100°C, 50°C to 80°C and 70°C to 80°C. In some embodiments, the solution is warmed to 45°C to 120°C and then held for a period of time to allow the dissolution and clarification of the compound of formula X; in some embodiments, the solution is warmed to 50°C to 100°C; in some embodiments, the solution is warmed to 50°C to 80°C; in some embodiments, the solution is warmed to about 50°C (e.g., 50 ±5°C); and in some embodiments, the solution is warmed to 70°C to 80°C. In some embodiments, the temperature T1 is about any of the following temperatures: 48°C, 50°C, 55°C, 60°C, 65°C, 70°C, 75°C, and 80°C, and can be maintained constant at the enumerated temperatures, and is also allowed to fluctuate within certain ranges, such as ±1°C, ±2°C, ±3°C, ±4°C, ±5°C, and the like. In some preferred embodiments, the temperature T1 is a constant temperature condition.

In some embodiments, in the step (b1), the solution is crystallized by cooling, volatilization, or anti-solvent addition.

In some embodiments, in the step (b1), the crystallization is by means of cooling crystallization, i.e., the crystallization is carried out by means of cooling crystallization. The cooling is in terms of a temperature relative to the temperature T1 in step (a1). In some embodiments, the solution is cooled down to temperature T2 and then held for a period of time to allow crystals to precipitate. The temperature T2 can be a constant temperature condition (maintained at a constant temperature) or a temperature range (maintained within a constant temperature range). In some embodiments, the temperature T2 is any temperature ranges selected from the following temperature ranges: -10°C to room temperature, 30°C to 20°C, 25°C to 15°C, 0°C to 5°C, 0°C to 4°C, below 0°C and 0°C to -5°C. In some embodiments, the temperature T2 is any temperature selected from the following temperature ranges: -10°C to room temperature, 30°C to 20°C, 25°C to 15°C, 0°C to 5°C, 0°C to 4°C, below 0°C, and 0°C to -5°C. In some embodiments, the solution is cooled to any temperature within the range from -10°C to room temperature; in some embodiments, the solution is cooled to room temperature; in some embodiments, the solution is cooled to 30°C to 20°C; in some embodiments, the solution is cooled to 25°C to 15°C; in some embodiments, the solution is cooled to 0°C to 5°C; in some embodiments, the solution is cooled to 0°C or below 0°C; and in some embodiments, the solution is cooled to 0°C to -5°C. In some embodiments, the temperature T2 is about any of the following temperatures: -5°C, -4°C, -2°C, 0°C, 5°C, 10°C, 15°C, 20°C, and 25°C, and can be maintained constant at the enumerated temperatures, and is also allowed to fluctuate within certain ranges, such as ±1°C, ±2°C, ±3°C, ±5°C, and the like. In some preferred embodiments, the temperature T2 is a constant temperature condition.

In some embodiments, the free alkali crystal form I is prepared by any of the following schemes:
(Ia) the solvent is acetonitrile, isopropanol, ethanol, ethyl acetate, or a 50% (v/v) ethanol/water solvent mixture, and the crystallization is cooling crystallization;
(Ib) the solvent is a 50% (v/v) ethanol/water solvent mixture and the crystallization is volatilizing crystallization; or
(Ic) the solvent is dimethylsulfoxide or N,N-dimethylacetamide, and the crystallization is anti-solvent crystallization.

In some embodiments, the compound of formula X is dissolved with acetonitrile, isopropanol, ethanol, ethyl acetate, or 50% ethanol to obtain a clarified solution, and the crystallization is carried out using cooling crystallization to obtain the free alkali crystal form I of the present application.

In some embodiments, the preparation method for the free alkali crystal form I comprises the steps of: dissolving the compound of formula X in isopropanol, ethanol, ethyl acetate, or a 50% (v/v) ethanol/water solvent mixture at 50±5°C to form a clarified solution; and cooling the solution to 0°C to 4°C and then held for a certain period of time to allow the crystals to precipitate. In some preferred embodiments, the solution is naturally cooled to room temperature and then left at 0°C to 4°C to precipitate solids.

In some embodiments, the preparation method for the free alkali crystal form I comprises the steps of: dissolving the compound of formula X in ethanol at 75±5°C to form a clarified solution; and cooling the solution to room temperature to precipitate crystals.

In some embodiments, the preparation method for the free alkali crystal form I comprises the steps of: dissolving the compound of formula X in ethanol at 70±5°C to form a clarified solution; cooling the solution to room temperature to precipitate crystals. In some preferred embodiments, the solution is cooled to room temperature by natural cooling.

In some embodiments, the preparation method for the free alkali crystal form I comprises the steps of: dissolving the compound of formula X in acetonitrile at 75±5°C to form a clarified solution; and cooling the solution to room temperature to precipitate crystals. In some preferred embodiments, the solution is cooled to room temperature by natural cooling.

In some embodiments, the compound of formula X is dissolved with 50% ethanol, and the crystallization is carried out by volatilizing crystallization to obtain the free alkali crystal form I.

In some embodiments, the preparation method for the free alkali crystal form I comprises the steps of: dissolving the compound of formula X in a 50% (v/v) ethanol/water solvent mixture to form a clarified solution; and volatilizing crystallizing the solution at room temperature. In some preferred embodiments, the solution is volatilized to crystallize by natural volatilization at room temperature.

In some embodiments, the preparation method for the free alkali crystal form I comprises the steps of dissolving the compound of formula X in dimethylsulfoxide or N,N-dimethyl acetamide to form a clarified solution; adding n-heptane to the solution to precipitate crystals.

In some embodiments, the compound of formula X is dissolved to clarification with dimethylsulfoxide or N,N-dimethylacetamide, and the crystallization is carried out by anti-solvent crystallization (or by means of an anti-solvent addition method) to obtain the free alkali crystal form I. In some of these embodiments, the anti-solvent used in the anti-solvent addition is n-heptane.

In some embodiments, the polymorph of the compound of formula X is the free alkali crystal form IV, and the crystallization is carried out by volatilization or anti-solvent addition.

The free alkali crystal form I obtained by the preparation method of the second aspect preferably conforms to any characterization method herein, including, but not limited to, an X-ray powder diffraction pattern, a combination of diffraction angle 2θ (°) characteristic peaks of the X-ray powder diffraction pattern, a differential scanning calorimetry curve, an endothermic peak of the differential scanning calorimetry curve, and a TGA-DSC plot.

In some embodiments, a preparation method for the free alkali crystal form IV is provided, comprising the steps of:
(a2) dissolving the compound of formula X in the presence of a solvent to form a clarified solution; and
(b2) crystallizing the solution by volatilization or anti-solvent addition.

In some embodiments, the solvent in the step (a2) is selected from acetonitrile, water, methanol, ethanol, isopropanol, acetone, ethyl acetate, tetrahydrofuran and a mixture thereof. In some embodiments, the solvent is acetone, acetonitrile, ethanol or a mixture thereof.

In some embodiments, the compound of formula X is dissolved to clarification with acetone and crystallized by volatilizing crystallization (e.g., slow volatilization) to obtain the free alkali crystal form IV.

In some embodiments, the compound of formula X is dissolved to clarification with ethanol or acetonitrile, and the crystallizing is carried out by anti-solvent addition to obtain the free alkali crystal form IV. In some of these embodiments, the anti-solvent used is n-heptane.

In some embodiments, in the step (b2), the solution is crystallized by slow volatilization or anti-solvent addition.

In some embodiments, the polymorph of a compound of formula X is the free alkali crystal form V, and the crystallization is carried out by volatilization. In some embodiments, the crystallization is carried out by slow volatilization.

In some embodiments, a preparation method for a free alkali crystal form V is provided, comprising the steps of:
(a3) dissolving the compound of formula X in the presence of a solvent to form a clarified solution; and
(b3) crystallizing the solution by volatilization.

In some embodiments, in step (a3), the solvent is ethanol.

In some embodiments, in the step (b3), the solution is crystallized by slow volatilization.

In some embodiments, the polymorph of the compound of formula X is the free alkali crystal form VI, and the crystallization is carried out by volatilization.

In some embodiments, a preparation method for a crystal form VI is provided, comprising the steps of:
(a4) dissolving the compound of formula X in the presence of a solvent to form a clarified solution; and
(b4) crystallizing the solution by volatilization.

In some embodiments, in step (a4), the solvent is acetonitrile, tetrahydrofuran, dimethylsulfoxide, or N,N-dimethylacetamide.

In some embodiments, in the step (b4), the solution is crystallized by slow volatilization.

In some embodiments, the polymorph of the compound of formula X is the free alkali crystal form VII, and the crystallization is carried out by anti-solvent addition.

In some embodiments, a preparation method for a free alkali crystal form VII is provided, comprising the steps of:
(a5) dissolving the compound of formula X in the presence of a solvent to form a clarified solution; and
(b5) crystallizing the solution by anti-solvent addition.

In some embodiments, in the step (a5), the solvent is methanol.

In some embodiments, the anti-solvent used in the step (b5) in the anti-solvent addition is n-heptane.

In the second aspect of the present application, there is also provided a preparation method for the polymorph of the pharmaceutically acceptable salt of the compound of formula X, comprising the steps of:
(a'0) mixing the compound of formula X with an acid in a solvent to form a solution; and
(b'0) crystallizing the solution.

The type of the acid for forming a salt with the compound of formula X is selected according to the type of the pharmaceutically acceptable salt.

The molar ratio of the compound of formula X to the acid ingredient of the pharmaceutically acceptable salt is controlled according to the amount of the acid. Preferably, an excess of acid is used.

In some embodiments, the acid is selected from: hydrochloric acid, sulfuric acid and hydrobromic acid.

In some embodiments, a monobasic acid is used and the molar ratio of the monobasic acid and the compound of formula X is 1.2:1.

In some embodiments, a dibasic acid is used and the molar ratio of the dibasic acid to the compound of formula X is 0.6:1.

In some embodiments, the polymorph of the pharmaceutically acceptable salt of the compound of formula X is the hydrochloride crystal form I, in which the acid is hydrochloric acid, and the crystallization is carried out by cooling, volatilization, or anti-solvent addition.

In some embodiments, the polymorph of the pharmaceutically acceptable salt of the compound of formula X is the hydrochloride crystal form I, in which the acid is hydrochloric acid, and the crystallization is carried out by slow cooling, slow volatilization, or anti-solvent addition.

In some embodiments, a preparation method for a hydrochloride crystal form I is provided, comprising the steps of:
(a6) mixing the compound of formula X and a dilute hydrochloric acid solution (e.g., a 1 M aqueous hydrochloric acid solution) in the presence of a solvent to form a solution; and
(b6) crystallizing the solution by cooling, volatilization or anti-solvent addition.

In some embodiments, the solvent in the step (a6) is selected from acetonitrile, water, methanol, ethanol, isopropanol, acetone, ethyl acetate, methyl tert-butyl ether, tetrahydrofuran, and a mixture thereof. In some embodiments, the solvent is isopropanol or acetone.

In some embodiments, the acid in the step (a6) is 1 M hydrochloric acid.

In some embodiments, the acid in the step (a6) is a 1 M aqueous hydrochloric acid solution.

In some embodiments, in the step (b6), the solution is crystallized by slow cooling, slow volatilization, or anti-solvent addition.

In some embodiments, in the step (b6), the crystallization is carried out by anti-solvent addition, and the anti-solvent used is n-heptane.

In some embodiments, the polymorph of the pharmaceutically acceptable salt of the compound of formula X is sulfate crystal form I, in which the acid is sulfuric acid, and the crystallization is carried out by cooling (preferably slow cooling) or anti-solvent addition.

In some embodiments, a preparation method for the sulfate crystal form I is provided, comprising the steps of:
(a7) mixing the compound of formula X and a dilute sulfuric acid solution (e.g., a 0.5 M aqueous sulfuric acid solution) in the presence of a solvent to form a solution; and
(b7) crystallizing the solution by cooling or anti-solvent addition.

In some embodiments, the solvent in the step (a7) is selected from acetonitrile, water, methanol, ethanol, isopropanol, acetone, ethyl acetate, methyl tert-butyl ether, tetrahydrofuran, and a mixture thereof. In some embodiments, the solvent is selected from ethanol, isopropanol, acetone, ethyl acetate and a mixture thereof.

In some embodiments, the acid in the step (a7) is 0.5 M sulfuric acid.

In some embodiments, the acid in the step (a7) is a 0.5 M aqueous sulfuric acid solution.

In some embodiments, in the step (b7), the solution is crystallized by slow cooling or anti-solvent addition.

In some embodiments, the crystallization in the step (b7) is carried out by anti-solvent addition, and the anti-solvent used is n-heptane.

In some embodiments, the polymorph of the pharmaceutically acceptable salt of the compound of formula X is hydrobromide crystal form I, in which the acid is hydrobromic acid, and the crystallization is carried out by cooling (preferably slow cooling).

In some embodiments, a preparation method for the hydrobromide crystal form I is provided, comprising the steps of:
(a8) mixing the compound of formula X and hydrobromic acid in the presence of a solvent to form a solution; and
(b8) crystallizing the solution by slow cooling.

In some embodiments, the solvent in the step (a8) is selected from acetonitrile, water, methanol, ethanol, isopropanol, acetone, ethyl acetate, methyl tert-butyl ether, tetrahydrofuran, and a mixture thereof. In some embodiments, the solvent is selected from ethanol, isopropanol, acetone, ethyl acetate and a mixture thereof.

In some embodiments, in the step (b8), the solution is crystallized by slow cooling.

In some embodiments, the preparation method for the polymorph further comprises the step of preparing the compound of formula X as follows: 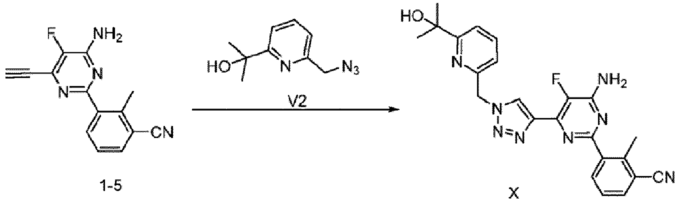 generating the compound of formula X by reaction of Compound **1-5** and Compound **V2** in an organic solvent.

Examples of the organic solvent include, but are not limited to: tetrahydrofuran (THF).

In some embodiments, the preparation method for the polymorph further comprises the step of preparing the Compound **1-5** as follows: 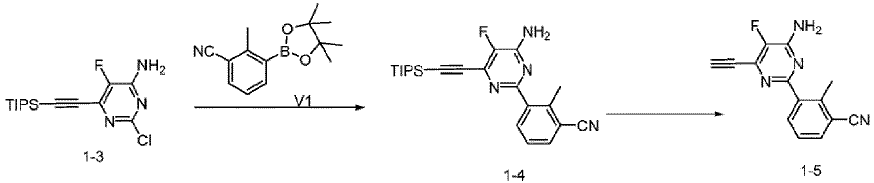 generating Compound **1-4** by reacting Compound **1-3** with Compound **V1**, and removing TIPS to generate the Compound **1-5;** wherein TIPS is triisopropylsilyl.

Those skilled in the art know how to remove TIPS, which will not be repeated in details here.

In a third aspect of the present application, there is provided a pharmaceutical composition comprising:
(a) any one of the polymorph (the polymorph of the compound of formula X or the pharmaceutically acceptable salt thereof) as described in the first aspect of the present application; and (b) a pharmaceutically acceptable carrier.

In a fourth aspect of the present application, there is provided use of the polymorph described in the first aspect of the present application (the polymorph of the compound of formula X or the pharmaceutically acceptable salt thereof), or the pharmaceutical composition described in the third aspect of the present application, in the manufacture of a medicament for prevention, treatment, or prophylaxis of a disease mediated by adenosine A_{2A} receptor, mediated by adenosine A_{2B} receptor, or co-mediated by adenosine A_{2A} receptor in conjunction with adenosine A_{2B} receptor.

In some embodiments, the compound of formula X ingredient acts as an A_{2A}/A_{2B} receptor antagonist.

In some embodiments, the disease is a tumor or an immune-related disease.

In some embodiments, the disease is a tumor. For the purposes of the present application, a tumor includes, but is not limited to, cancer.

In some embodiments, the disease is cancer.

In some embodiments, the tumor is selected from prostate cancer, colon cancer, rectal cancer, pancreatic cancer, cervical cancer, gastric cancer, endometrial cancer, brain cancer, liver cancer, bladder cancer, ovarian cancer, testicular cancer, head cancer, neck cancer, melanoma, basal carcinoma, mesothelial endodermal cancer, leukemia, esophageal cancer, breast cancer, muscular carcinoma, connective tissue carcinoma, small-cell lung cancer, non-small-cell lung cancer, adrenal gland cancer, thyroid cancer, kidney cancer, and bone cancer; and can be further selected from malignant glioma, mesothelioma, renal cell carcinoma, gastric cancer, sarcoma, choriocarcinoma, basal cell carcinoma of the skin, and seminomas of the testes.

In some embodiments, the disease is an immune-related disease.

In some embodiments, the immune-related disease is selected from rheumatoid arthritis, renal failure, lupus, asthma, psoriasis, colitis, pancreatitis, allergies, fibrosis, anemic fibromyalgia, Alzheimer's disease, congestive heart failure, stroke, aortic stenosis, atherosclerosis, osteoporosis, Parkinson's disease, infections, Crohn's disease, ulcerative colitis, allergic contact dermatitis and other eczemas, systemic sclerosis and multiple sclerosis.

In a fifth aspect of the present application, there is provided a method of prevention, treatment, or prophylaxis of a disease mediated by adenosine A_{2A} receptor, mediated by adenosine A_{2B} receptor, or co-mediated by adenosine A_{2A} receptor in conjunction with adenosine A_{2B} receptor comprising administering to the desired patient a therapeutically effective amount of the polymorph described in the first aspect of the present application (the polymorph of the compound of formula X or the pharmaceutically acceptable salt of the compound of formula X), or the pharmaceutical composition of the third aspect of the present application.

It should be understood that, within the scope of the present application, each of the above technical features of the present application and each of the technical features described hereinafter (including, but not limited to, the examples) may be combined with each other in any suitable manner so as to constitute a new or preferred technical solution, as long as it can be used for implementing the present application. Due to space limitation, we will not repeat them herein. The compound of formula X of the present application has good relevant pharmacological activities.

Within the tumor microenvironment, two adenosine receptors, A_{2A} and A_{2B}, are widely expressed in immune cells and have strong immunosuppressive functions. The increase of extracellular adenosine concentration is one of the important mechanisms of action for immune escape of tumor cells, and the concentration level is determined by the level of ATP and the expression level of CD39 and CD73 together. The increase in extracellular adenosine concentration is associated with the release of large amounts of ATP from cell death or hypoxia in the tumor microenvironment, and the concentration can reach 10 to 20 times higher than that of normal tissue. Binding of adenosine to adenosine receptors in the tumor microenvironment can inhibit anti-tumor responses, such as inhibiting CD8+ T cell function, enhancing of immunosuppressive regulatory T cell function, and inhibiting antigen-presenting cell function via dendritic cells. Some studies have shown that binding to the A_{2A} receptor can also inhibit the tumor killing effects of natural killer cells. Some further studies have shown that A_{2A} adenosine receptor antagonists can increase the viability and killing ability of dendritic antigen-presenting cells, T cells, and natural killer cells, inhibit the function of regulatory T cells (T-regs), bone marrow-derived suppressor cells (MDSCs), and tumor-associated macrophages (TAMs), eliminate tumor immune tolerance, and promote the development of the immune response to tumors, which in turn leads to the inhibition of tumor growth and prolongation of mouse survival. In addition, A_{2B} receptor has been reported to promote tumor migration in murine melanoma and triple-negative breast cancer models, and thus A_{2B} receptor antagonists are also effective targets for tumor therapy. Therefore, blocking adenosine signaling pathway activation to reduce or release immunosuppression and to enhance the anti-tumor function of immune cells (especially T cells) is considered to be one of the effective means of tumor therapy.

The polymorphs of the compound of formula X and the pharmaceutically acceptable salt thereof (i.e., the polymorph of the compound of formula X and the polymorph of the pharmaceutically acceptable salt of the compound of formula X) of the present application have good stability, and thus have the potential for further medicinal development.

### BRIEF DESCRIPTION OF THE DRAWINGS

FIG. 1 shows an X-ray powder diffraction (XRPD) pattern of free alkali crystal form I in one embodiment of the present application, using Cu-Kα radiation, with angle 2θ (°) as the horizontal coordinate, intensity as the vertical coordinate, and counts as the vertical coordinate unit.
FIG. 2 shows a TGA-DSC (Thermogravimetric Analysis-Differential Scanning Calorimetry) plot of the free alkali crystal form I in one embodiment of the present application, with the temperature (°C) as the horizontal coordinate, Change In Mass (%) as the left vertical coordinate, and the Heat Flow (Normalized) Q (W/g) as the right vertical coordinate.
FIG. 3 shows the Dynamic Vapor Sorption (DVS) plot of free alkali crystal form I in one embodiment of the present application, with Target RH (%) as the horizontal coordinate and Change In Mass (%)-Ref as the vertical coordinate.
FIG. 4 shows an X-ray powder diffraction (XRPD) pattern of the free alkali crystal form IV in one embodiment of the present application, using Cu-Kα radiation, with angle 2θ (°) as the horizontal coordinate, intensity as the vertical coordinate, and counts as the vertical coordinate unit.
FIG. 5 shows a differential scanning calorimetry (DSC) plot of free alkali crystal form IV in one embodiment of the present application, with temperature (°C) as the horizontal coordinate and Heat Flow (Normalized) Q (W/g) as the vertical coordinate.
FIG. 6 shows an X-ray powder diffraction (XRPD) pattern of free alkali crystal form V in one embodiment of the present application, using Cu-Kα radiation, with angle 2θ (°) as the horizontal coordinate, intensity as the vertical coordinate, and counts as the vertical coordinate unit.
FIG. 7 shows a differential scanning calorimetry (DSC) plot of free alkali crystal form V in an embodiment of the present application, with temperature (°C) as the horizontal coordinate and Heat Flow (Normalized) Q (W/g) as the vertical coordinate.
FIG. 8 shows an X-ray powder diffraction (XRPD) pattern of free alkali crystal form VI in one embodiment of the present application, using Cu-Kα radiation, with angle 2θ (°) as the horizontal coordinate, intensity as the vertical coordinate, and counts as the vertical coordinate uni.
FIG. 9 shows a differential scanning calorimetry (DSC) plot of free alkali crystal form VI in one embodiment of the present application, with temperature (°C) as the horizontal coordinate and Heat Flow (Normalized) Q (W/g) as the vertical coordinate.
FIG. 10 shows an X-ray powder diffraction (XRPD) pattern of free alkali crystal form VII in one embodiment of the present application, using Cu-Kα radiation, with angle 2θ (°) as the horizontal coordinate, intensity as the vertical coordinate, and counts as the vertical coordinate unit.
FIG. 11 shows a differential scanning calorimetry (DSC) plot of free alkali crystal form VII in one embodiment of the present application, with temperature (°C) as the horizontal coordinate and Heat Flow (Normalized) Q (W/g) as the vertical coordinate.
FIG. 12 shows a comparison of XRPD patterns of free alkali crystal form I at 40°C/75% RH in one embodiment of the present application, with angle 2θ (°) as the horizontal coordinate, intensity as the vertical coordinate, and counts as the vertical coordinate unit.
FIG. 13 shows an X-ray powder diffraction (XRPD) pattern of hydrochloride crystal form I in one embodiment of the present application, using Cu-Kα radiation, with angle 2θ (°) as the horizontal coordinate, intensity as the vertical coordinate, and counts as the vertical coordinate unit.
FIG. 14 shows a Differential Scanning Calorimetry (DSC) plot of hydrochloride crystal form I in one embodiment of the present application, with temperature (°C) as the horizontal coordinate and Heat Flow (Normalized) Q (W/g) as the vertical coordinate.
FIG. 15 shows an X-ray powder diffraction (XRPD) pattern of sulfate crystal form I in one embodiment of the present application, using Cu-Kα radiation, with the angle 2θ (°) as the horizontal coordinate, the intensity as the vertical coordinate, and counts as the vertical coordinate unit.
FIG. 16 shows a differential scanning calorimetry analysis (DSC) plot of sulfate crystal form I in one embodiment of the present application, with temperature (°C) as the horizontal coordinate and Heat Flow (Normalized) Q (W/g) as the vertical coordinate.
FIG. 17 shows an X-ray powder diffraction (XRPD) pattern of hydrobromide crystal form I in one embodiment of the present application, using Cu-Kα radiation, with angle 2θ (°) as the horizontal coordinate, the intensity as the vertical coordinate, and counts as the vertical coordinate unit.
FIG. 18 shows a comparison of the XRPD patterns of hydrochloride crystal form I in one embodiment of the present application at 60°C, and 40°C/75% RH, respectively, with Two-Theta (2θ) as the horizontal coordinate and deg (°) as the horizontal coordinate unit, and with Intensity as the vertical coordinate and counts as the vertical coordinate unit.
FIG. 19 shows a comparison of the XRPD patterns of sulfate crystal form I of an embodiment of the present application at 60°C, and 40°C/75% RH, respectively, with Two-Theta (2θ) as the horizontal coordinate, and deg (°) as the horizontal coordinate unit, and with Intensity as the vertical coordinate and counts as the vertical coordinate unit; wherein, "HY80****" is the sample number of the sulfate compound.
FIG. 20 shows an infrared spectrum of free alkali crystal form I in an embodiment of the present application, with wavenumber (cm⁻¹) as the horizontal coordinate, and Transmittance as the vertical coordinate.

### DETAILED DESCRIPTION

The present application is described in further detail below in conjunction with a number of accompanying drawings, embodiments and examples. It should be understood that these embodiments and examples are only used to illustrate the present application and are not intended to limit the scope of the present application, and that they are provided for the purpose of enabling a more thorough and comprehensive understanding of the disclosure of the present application. It should also be understood that the present application can be realized in many different forms, and is not limited to the embodiments and examples described herein, and the person skilled in the art can make various changes or modifications without departing from the connotation of the present application, and the equivalent forms obtained also fall within the protection scope of the present application. Furthermore, in the description below, a great deal of detail is given in order to provide a fuller understanding of the present application, and it should be understood that the present application can be implemented without one or more of these details.

Unless otherwise defined, all technical and scientific terms used herein have the same meaning as commonly understood by those skilled in the art belonging to the present application. The terms used herein in the specification of the present application are used only for the purpose of describing embodiments and examples and are not intended to limit the present application.

### Terminology

Unless otherwise stated or there is a contradiction, terms or phrases used herein have the following meanings.

For the purposes of the present application, the choices of "and/or" and "or/and" include any one of two or more relevant listed items, as well as any and all combinations of the relevant listed items, including combinations of any two of the relevant listed items, any more of the relevant listed items, or all of the relevant listed items. It is to be noted that when connecting at least three items with combinations of at least two conjunctions selected from "and/or" and "or/and", it is to be understood that, in the present application, the technical solution undoubtedly includes technical solutions that are all connected by "logical and" and also undoubtedly includes technical solutions that are all connected by "logical or". For example, "A and/or B" includes A, B and A+B. For example, the technical solution "A, and/or, B, and/or, C, and/or, D" includes any one of A, B, C, and D (i.e., technical solutions connected by "logical or"), as well as any and all combinations of A, B, C, D, i.e., any two or three of A, B, C, and D, and also the combination of A, B, C, and D (i.e., technical solutions connected by "logical and").

In the present application, "a combination thereof", "any combination thereof", "any combination manner thereof", and the like include all suitable combinations of any two or more of the listed items.

In the present application, the "suitable" referred to in "suitable combination manner", "suitable manner", "any suitable manner", and the like, is based on being able to implement the technical solutions of the present application, solve the technical problems of the present application, and realize the expected technical effects of the present application.

In the present application, "preferred", "preferably", "better", and "appropriate" are only used to describe an embodiment or example with better effect, and should be understood as not constituting a limitation on the protection scope of the present application.

In the present application, "optionally", and "optional" means either with or without, i.e., either of the two parallel solutions of "with" or "without". If there are more than one "optional" in a technical solution, each "optional" is independent of the other, if not otherwise specified and if there are no contradictions or mutual constraints.

In the present application, the terms "first", "second", "third", "fourth", etc., in "first aspect", "second aspect", "third aspect", "fourth aspect", etc., are used for descriptive purposes only and are not to be construed as indicating or implying relative importance or quantity, or as implicitly specifying the importance or quantity of the technical features indicated. Moreover, "first", "second", "third", "fourth" and the like only serve the purpose of non-exhaustive enumeration, and it should be understood that they do not constitute a closed-ended limitation of quantity.

In the present application, among the technical features described in an open-ended manner, a closed technical solution comprising the enumerated features is included, and an open-ended technical solution comprising the enumerated features is also included.

The present application relates to numerical intervals (i.e., ranges of values), wherein, if not specifically stated, the optional distribution of values is considered continuous within the numerical intervals and includes the two numerical endpoints (i.e., the minimum value and the maximum value) of the range of values, as well as each of the numerical values between these two numerical endpoints. Unless otherwise noted, when the numerical range is directed only to integers within that numerical range, the two endpoint integers of that numerical range are included, as well as each integer between the two endpoints. In addition, when multiple ranges are provided to describe the feature or characteristic, the ranges may be combined. In other words, unless otherwise indicated, the ranges disclosed herein should be understood to include any and all sub-ranges subsumed therein.

In the present application, "M", "nM", "µM" and "mM" are used to denote concentrations that are equivalent to mol/L, nmol/L, µmol/L and mmol/L, respectively, if not otherwise specified.

In the present application, "have/has/having" is open-ended. For example, "having peaks at certain positions of the plot" indicates that peaks are present at those positions of the plot and does not preclude peaks from being present at other positions of the plot.

The inventors, after extensive and intensive research, discovered the compound of formula X, which was found to have high inhibitory activity against adenosine A_{2A} receptor, adenosine A_{2B} receptor, or both of these receptors. On this basis, a series of polymorphs of the compound of formula X and pharmaceutically acceptable salts thereof were accidentally discovered through further research, which not only have better stability, but also have better *in vivo* and *in vitro* relevant pharmacological activities, and thus have the possibility of being further developed into medicaments.

### Compound of the present application

In the present application, the compound of formula X is 3-(4-amino-5-fluoro-6-(1-((6-(2-hydroxypropan-2-yl)pyridin-2-yl)methyl)-1H-1,2,3-triazol-4-yl)pyrimidin-2-yl)-2-methylbenzonitrile, having the structure shown in formula (X),

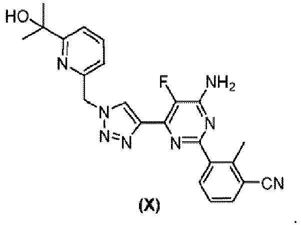

The compound of formula X is structurally a pyrimidine derivative. The compound of formula X is an A_{2A}/A_{2B} dual receptor antagonist, which can block the activation of both receptors A_{2A} and A_{2B}, and has a high inhibitory activity on adenosine A_{2A} receptor, adenosine A_{2B} receptor, or both receptors. From mechanism aspect, the compound of formula X regulates different immune cell populations, integrally blocks the immunosuppressive effect brought by adenosine within the microenvironment, which has far-reaching clinical application value for tumor treatment and can be used in the treatment of tumor-related diseases, immune-related diseases, or tumor and immune-related diseases.

In the present application, "A_{2A}/A_{2B} receptor antagonist" denotes both an A_{2A} receptor antagonist and an A_{2B} receptor antagonist, which can block activation of both A_{2A} and A_{2B} receptors.

The present application also includes polymorphs of the compound of formula X and the pharmaceutically acceptable salt thereof, wherein the pharmaceutically acceptable salt is not specifically limited but is preferably selected from hydrochloride, sulfate and hydrobromide salts.

In the present application, polymorphs include, but are not limited to, polymorphs of free alkalis, and polymorphs of pharmaceutically acceptable salts.

For the purposes of the present application, polymorphs of the compound of formula X, when not specifically limited, refer to polymorphs of the free alkali of the compound of formula X, including, but not limited to, free alkali crystal form I, free alkali crystal form IV, free alkali crystal form V, free alkali crystal form VI, and free alkali crystal form VII in the present application.

In the present application, polymorphs of the compound of formula X and the pharmaceutically acceptable salts thereof include, but are not limited to, free alkali crystal form I, free alkali crystal form IV, free alkali crystal form V, free alkali crystal form VI, free alkali crystal form VII, hydrochloride crystal form I, sulfate crystal form I, and hydrobromide crystal form I of the compound of formula X.

In the present application, the type I crystal of the compound of formula X, and the free alkali crystal form I of the compound of formula X have the same meaning and can be used interchangeably. If not specifically limited, the free alkali crystal form I in the present application refers to the crystal form I of the compound of formula X.

In the present application, the type IV crystal of the compound of formula X, and the free alkali crystal form IV of the compound of formula X have the same meaning and can be used interchangeably. If not specifically limited, the free alkali crystal form IV in the present application refers to the crystal form IV of the compound of formula X.

In the present application, the type V crystal of the compound of formula X, and the free alkali crystal form V of the compound of formula X have the same meaning and can be used interchangeably. If not specifically limited, the free alkali crystal form V in the present application refers to the crystal form V of the compound of formula X.

In the present application, the type VI crystal of the compound of formula X, and the free alkali crystal form VI of the compound of formula X have the same meaning and can be used interchangeably. If not specifically limited, the free alkali crystal form VI in the present application refers to the crystal form VI of the compound of formula X.

In the present application, the type VII crystal of the compound of formula X, and the free alkali crystal form VII of the compound of formula X have the same meaning and can be used interchangeably. If not specifically limited, the free alkali crystal form VII in the present application refers to the crystal form VII of the compound of formula X.

In the present application, the type I crystal of the hydrochloride of the compound of formula X, and the hydrochloride crystal form I of the compound of formula X have the same meaning and can be used interchangeably. If not specifically limited, the hydrochloride crystal form I in the present application refers to the hydrochloride crystal form I of the compound of formula X.

In the present application, the type I crystal of the sulfate of the compound of formula X, and the sulfate crystal form I of the compound of formula X have the same meaning and can be used interchangeably. If not specifically limited, sulfate crystal form I in the present application refers to the sulfate crystal form I of the compound of formula X.

In the present application, the type I crystal of the hydrobromide of the compound of formula X, and the hydrobromide crystal form I of the compound of formula X have the same meaning and can be used interchangeably. If not specifically limited, hydrobromide crystal form I in the present application refers to hydrobromide crystal form I of the compound of formula X.

As used herein, "medicament" includes any agent, compound, composition or mixture that provides a physiological, pharmacological or physiological and pharmacological effect *in vivo* or *in vitro,* often providing a beneficial effect. There is no particular limitation to the extent that the "medicament" produces physiological, pharmacological or physiological and pharmacological effects *in vivo,* which may be systemic or localized. There is no particular limitation on the activity of the "medicament", which may be an active ingredient that interacts with other ingredients or an inert ingredient that does not interact.

As used herein, "therapeutically effective amount" means an amount of a compound of the present application that will elicit a biological or medical response in an individual, such as an amount of a compound of the present application that will result in a physiologically, pharmacologically, or physiologically and pharmacologically positive effect for the individual, the physiologically, pharmacologically, or physiologically and pharmacologically positive effect including, but not limited to, reducing or inhibiting enzyme or protein activity or ameliorating a symptom, alleviating a condition, slowing or delaying a disease process, or preventing a disease.

As used herein, "pharmaceutically acceptable" means a ligand, material, composition, or dosage form that is suitable for administration to a patient within the bounds of reasonable medical judgment and that is commensurate with a reasonable benefit/risk ratio.

As used herein, "pharmaceutically acceptable carrier" should in principle be non-toxic and inert. The form of the "pharmaceutically acceptable carrier" is not specifically limited, and includes, but is not limited to, solid, semi-solid, liquid, and the like. The pharmaceutically acceptable carrier should be compatible with a patient, the patient being preferably a mammal, more preferably a human. One of the roles of the pharmaceutically acceptable carrier is to be suitable for delivering an active reagent to a target without terminating the activity of the reagent. As used herein, the term "pharmaceutically acceptable carrier" includes buffers, sterile water for injection, solvents, dispersing media, coatings, anti-bacterial and antifungal agents, isotonic and absorption delaying agents, and the like, which are compatible with medicament administration. Each carrier must be "pharmaceutically acceptable" in the sense that it is compatible with other ingredients of the formulation and is not harmful to the patient.

As used herein, "pharmaceutically acceptable salt" means a salt suitable for use as a medicament formed by any of the compounds in the indicated structures with an acid or base. In the present context, it refers primarily to a salt suitable for use as a medicament formed by a compound of formula X with an acid suitable.

As used herein, "patient" refers to an animal, preferably a mammal, more preferably a human. The term "mammal" refers primarily to warm-blooded vertebrate mammals, including, but not limited to, cats, dogs, rabbits, bears, foxes, wolves, monkeys, deer, mice, pigs, cows, sheep, horses, and humans.

The modes of administration of the compounds, polymorphs, pharmaceutical compositions, or agents, etc., are not specifically limited in the present application. Representative modes of administration include, but are not limited to: oral, intratumoral, rectal, parenteral (intravenously, intramuscularly, or subcutaneously) injections, and topical administration.

As used herein, "treatment" refers to the alleviation, slowing of progression, attenuation, prevention, or maintenance of an existing disease or condition (e.g., cancer). Treatment also includes curing, preventing the progression of, or mitigating to some degree one or more symptoms of a disease or condition.

In the present application, "prevention and/or treatment" means prevention, treatment, or prophylaxis. In this case, "prophylaxis" means both prevention and treatment.

In the present application, "using Cu-Kα radiation" means that the corresponding patterns are obtained using Kα-ray of the Cu target for detection, and when other methods are used for detection, the diffraction peaks may have deviations within the acceptable range of the field, which should not be construed as a limitation of the present application.

### Polymorph

Solids exist in amorphous, crystal or semi-crystal form. For small molecule (preferably with molecular weights up to 1000 Da) organic compounds, they exist mainly in amorphous or crystal form. In the case of the crystal form, the molecules are localized within the three-dimensional lattice compartments. When a compound is crystallized from a solution or slurry, it can crystallize in different spatial point arrangements (a property known as "polymorph phenomenon") to form crystals with different crystal forms, and those different crystal forms are called "polymorphs". Different polymorphs of a given material may differ from each other in terms of one or more physical properties (e.g., solubility, rate of dissolution, true specific gravity, crystal shape, mode of accumulation, mobility, and solid-state stability).

### Crystallization

Production-scale crystallization can be accomplished by manipulating the solution such that the solubility limit of the compound of interest is exceeded. This can be accomplished by a variety of methods, for example, dissolving the compound at a relatively high temperature and then cooling the solution below the saturation limit; or reducing the volume of the liquid by boiling, by evaporation at atmospheric pressure, by vacuum drying, or by some other methods; reducing the solubility of the compound of interest by adding an anti-solvent, or by adding a poor solvent in which the compound has a low solubility, or by adding mixtures of such anti-solvents or poor solvents. Another optional method is to adjust the pH to reduce the solubility. A detailed description of crystallization can be found in Crystallization, Third Edition, J W Mullens, Butterworth-Heineman Ltd. 1993, ISBN 0750611294.

### Identification and properties of crystal forms

In the present application, crystal forms of the compound of formula X were prepared, the properties of which were also investigated using a variety of means and apparatus including, but not limited to, the following.

### X-ray powder diffraction (XRPD)

The method of X-ray powder diffraction (XRPD) for the determination of crystal forms is known in the art. XRPD is a common means of identifying crystal forms because it can detect information such as changes in crystal form, crystallinity, and the state of crystalline structure. The position of the peaks in the XRPD pattern is mainly dependent on the structure of the crystal form. The measurements of the 2θ (Two-theta) in XRPD pattern may differ slightly between different instruments, and thus the 2θ values of characteristic peaks in XRPD pattern should not be regarded as absolute. Depending on the condition of the instrument used for the test in the present application, the diffraction peaks are allowed to have a certain error (e.g., ±0.2°). It can be understood that the error range is not absolute for different testing instruments and testing conditions. The crystal form of the compound of formula X of the present application has a specific crystalline morphology with specific characteristic peaks in the XRPD pattern.

### Differential Scanning Calorimetry (DSC)

Differential Scanning Calorimetry (DSC), also known as "Differential Calorimetry Scanning Analysis", is a technique that measures the relationship between the energy difference between the test material and the reference material and the temperature during a heating process. The positions, shapes and numbers of peaks on the DSC plot are related to the nature of the material, and therefore can be used to qualitatively identify the material. The method is commonly used in the art to detect a variety of parameters such as the phase transition temperature, glass transition temperature, and reaction heat of a material. The peak positions in the DSC plot may vary slightly between different instruments, so the value of the peak position of the DSC endothermic peak in the DSC plot cannot be considered as absolute. Depending on the condition of the instrument used in the test of the present application, the value of the experimental error or difference is also not absolute, and may be less than or equal to 5°C, or less than or equal to 4°C, or less than or equal to 3°C, or less than or equal to 2°C, or less than or equal to 1°C.

### Thermogravimetric Analysis (TGA)

TGA is a technique for determining the change in mass of a material as a function of temperature under program control, which is suitable for checking the loss of solvent in crystals or the process of sublimation and decomposition of samples, and can be used to speculate on the presence of crystal water or crystal solvent in crystals. The change in mass shown by the TGA curve depends on a number of factors, such as the preparation of the sample and the instrumentation; the change in mass as detected by the TGA varies slightly from instrument to instrument. Depending on the condition of the instrumentation used in the tests of the present application, the error in the mass change is not absolute, and a certain error (e.g., ±0.1%) is allowed.

In the present application, "the X-ray powder diffraction pattern has a peak at a specific diffraction angle 2θ (°)", wherein the peak refers to a diffraction peak, and the foregoing statement indicates that the peak value of the peak is within the indicated range of values, the indicated point of values, the neighborhood of the indicated range of values, or the neighborhood of the indicated point of values. Due to differences in measurement factors such as a measurement instrument and a measurement condition, the position of a certain peak or certain peaks in the X-ray powder diffraction pattern actually obtained may be slightly shifted. That is, there may be a slight difference between the combination of characteristic peaks or the X-ray powder diffraction pattern indicated in the present application. However, it is to be understood that it is possible for a person skilled in the art to be able to discern as a whole whether the combination of characteristic peaks or the X-ray powder diffraction patterns which differ slightly can be substantially recognized as consistent with the crystal form described in the present application. Accordingly, those which are substantially recognized as being consistent with the crystal form of the invention should be considered to be within the protection scope of the present application. For example, "X-ray powder diffraction pattern has a peak at a diffraction angle of 16.63°±0.2°" means that the value of the peak may be located within or near the range of 16.63°±0.2°, as long as it does not interfere with the identification of the crystal form as a whole. In addition, the expression "±0.2°" here only indicates the error in the position of the peak at the diffraction angle, and has nothing to do with other factors such as the peak shape and width of the peak.

In the present application, the term "substantially" in the expression "the X-ray powder diffraction pattern is substantially as characterized by the particular pattern" should be interpreted similarly, and as long as it is possible to determine that a certain X-ray powder diffraction pattern in its entirety is substantially the same as the X-ray powder diffraction pattern described in the present application, it should be considered to fall within the protection scope of the present application.

It is to be understood that the Differential Scanning Calorimetry (DSC) curves in the present application and the positions of the endothermic peaks shown therein should be similarly construed as allowing for slight discrepancies between a value or a range of values or a spectrum disclosed in the present application, and as long as it can be determined as a whole that some or all of the positions of the endothermic peaks in the DSC curves, or the entire curves, are substantially consistent with the present application, then the curves should be considered to fall within the protection scope of the present application.

Understandably, a similar understanding can be given to other spectra that characterize the type of crystallization (e.g., infrared spectra).

The term "dissolving to obtain a clarified solution" as used in the present application refers to the complete dissolution of the compound of formula X or a pharmaceutically acceptable salt thereof in a solvent.

The term "in the presence of a solvent" as used in the present application means that the system used to dissolve to clarification the compound or salt thereof includes a solvent and also allows for the presence of soluble and insoluble ingredients, or both soluble and insoluble ingredients, which are not solvents. The expression "in the presence of a solvent" described in the present application includes at least the technical solution of "in a solvent". Moreover, "in a solvent" is also an open-ended description, including a closed-ended case in which only a "solvent" is used for dissolving to clarification, as well as an open-ended case in which a co-solvent component is also present in the "solvent".

The term "cooling" as used in the present application refers to a method in which a compound of formula X or a pharmaceutically acceptable salt thereof is dissolved to clarification with a solvent at a relatively high temperature and then cooled to a certain temperature to obtain crystals.

The term "volatilizing" as used in the present application refers to a method of volatilizing a solution containing a compound of formula X or a pharmaceutically acceptable salt thereof at a certain temperature to volatilize the solvent to obtain crystals.

"Anti-solvent addition" as described herein refers to a method of precipitating crystals from a solution containing a compound of formula X or a pharmaceutically acceptable salt thereof by adding another suitable solvent. The suitable solvent may be an anti-solvent, a poor solvent or a combination thereof. In some embodiments, an anti-solvent is preferred.

If salt formation is expected to occur simultaneously with crystallization, and if the salt is less soluble than the feedstock in the reaction medium, the addition of an appropriate acid or base can lead to direct crystallization of the desired salt. Similarly, the completion of a synthetic reaction in a medium in which the final desired form is less soluble than the reactants can lead to direct crystallization of the final product.

Optimization of crystallization may include inoculation of the crystallization medium with crystals of a desired form as a crystalline seed. Additionally, many crystallization methods may use a combination of the above strategies. One embodiment is to dissolve the compound of interest in a solvent at high temperatures, followed by the addition of an appropriate volume of anti-solvent in a controlled manner to bring the system just below the saturation level. At this point, the desired form of crystalline seed can be added (and the integrity of the crystalline seed is maintained) and the system can be cooled to complete crystallization.

As used herein, the term "room temperature" generally refers to 4°C to 30°C, and may further refer to 20°C ± 5°C. In some embodiments of the present application, room temperature refers to 20°C to 30°C.

The temperature parameters in the present application, if not specifically limited, are allowed to be both thermostatically treated and to vary within a certain temperature interval. It should be understood that the thermostatic treatment allows for temperature fluctuations within the accuracy of the instrument control.

In the present application, it is permissible to use "a mixture thereof" to denote a mixture of two or more of the listed materials. For example, "a mixture thereof" in "the solvent is selected from acetonitrile, water, methanol, ethanol, isopropanol, acetone, ethyl acetate, 50% (v/v) ethanol/water solvent mixtures, dimethylsulfoxide, N,N-dimethylacetamide, tetrahydrofuran, and a mixture thereof" indicates a mixture of the aforementioned solvents formed in any suitable manner, firstly, that the resulting mixture should still be usable as a solvent, and secondly, that it is understood to refer only to solvent mixtures that can be realized.

In the present application, "% (v/v)" denotes a volume percentage. For example, "50% (v/v) ethanol/water solvent mixture" represents a mixture of ethanol and water in a 1:1 ratio by volume.

For the purposes of the present application, 50% ethanol, when not specifically limited, refers to a solvent mixture formed from anhydrous ethanol and water in a 1:1 ratio by volume, or it may be abbreviated as a 50% (v/v) ethanol/water solvent mixture.

### Identification and characterization of polymorphs

In the present application, after preparing polymorphs of the compound of formula X, their characteristics were investigated using a variety of means and apparatus including, but not limited to, the following.

### X-ray powder diffraction

The polymorphs of the compound of formula X and the pharmaceutically acceptable salts thereof of the present application have a specific crystalline morphology with specific characteristic peaks in the X-ray powder diffraction (XRPD) pattern. As one of the embodiments, the XRPD patterns in some embodiments of the present application were acquired on an Equinox 3000S/N X-ray powder diffraction analyzer with the XRPD parameters shown in Table 9.

**Table 9. XRPD test parameters in some embodiments of the present application.**

| Parameter term | XRPD Parameters |
|---|---|
| X-ray source | Cu K (λ=1.54056 Angstrom) |
| Light Pipe Setting | 40 kV, 30 mA |
| Detector | PSD |
| Scanning range (2Theta) | 0° to 120° |
| Scanning step (2Theta) | 0.03° |
| Scanning speed | 1 sec/step |

2Theta, also known as 2θ, is measured in unit of "°", which can also be written as "deg".

In an X-ray powder diffraction pattern, the position of each peak is determined by 2θ (°). It is understood that different instruments, different conditions, or different instruments and conditions may lead to slight differences in the result data, including variations in the positions and relative intensities of the peaks. It should be understood by those skilled in the art that elements such as peaks at low diffraction angle and intensities thereof, peak shape integrity, and the like are relatively more informative when determining a crystal form based on the XRPD pattern. In the present application, the applicant, after further research, found that when testing for the XRPD pattern using the apparatus described above, it is possible that strong background peaks of a blank metal disk may appear near the 2θ (°) values of 37.4° and 43.6° at high diffraction angle. For example, the peak near 37° in FIG. 4 is presumed to be generated by blank metal disk (e.g., peaks at 37.76° in Table 2), the peak near 37° and the peak near 43° in FIG. 6 are presumed to be generated by blank metal disk (e.g., the peaks at 37.43° and 43.60° in Table 3), the peak near 37° in FIG. 8 is presumed to be a peak generated by blank metal disk (e.g., the peak at 37.42° in Table 4), and the peak near 37° and the peak near 43° in FIG. 10 are presumed to be peaks generated by blank metal disk (e.g., the peaks at 37.40° and 43.55° in Table 5). The intensity division of the peaks simply reflects the approximate size of the peaks at each position. In the present application, the diffraction peaks of each crystal form are analyzed by using the diffraction peak with the highest peak height in the XRPD pattern obtained by the aforementioned method as the base peak, the relative intensity of which is defined as 100% as I₀ (e.g., in some embodiments of the present application, the peak with 2θ (°) value of 21.41 in the XRPD pattern of the free alkali crystal form I is the base peak, the peak with 2θ (°) value of 16.46 in the XRPD pattern of the free alkali crystal form IV is the base peak, the peak with 2θ (°) value of 14.35 in the XRPD pattern of free alkali crystal form V is the base peak, the peak with 2θ (°) value of 24.53 in the XRPD pattern of free alkali crystal form VI is the base peak, the peak with 2θ (°) value of 37.40 in the XRPD pattern of free alkali crystal form VII is the base peak, the peak with 2θ (°) value of 22.58 in the XRPD pattern of hydrochloride crystal form I is the base peak, the peak with a 2θ (°) value of 23.48 in the XRPD pattern of sulfate crystal form I is the base peak, and the peak with a 2θ (°) value of 23.51 in the XRPD pattern of hydrobromide crystal form I is the base peak), and for each of the other peaks, the ratio of the peak height thereof to that of the base peak is the relative intensity I/I₀. The division of the relative intensity of each peak is defined as shown in Table 10 in the present application.

**Table 10. Definition of the division of the relative intensity of each peak of the XRPD pattern in the present application.**

| Relative Intensity I/I₀ (%) | Definition |
|---|---|
| 50 to100 | VS (very strong) |
| 25 to 50 | S (strong) |
| 10 to 25 | M (medium) |
| 1 to 10 | W (weak) |

### Differential Scanning Calorimetry (DSC)

As one of the embodiments, DSC plots in some embodiments of the present application were collected on a DISCOVERY DSC25 differential scanning calorimeter with the test parameters shown in Table 11.

**Table 11. DSC test parameters in some embodiments of the present application.**

| Parameter term | DSC Parameters |
|---|---|
| Method | Linear warming |
| Sample plate | Aluminum Plate, Gland |
| Temperature range | 25°C to 300°C |
| Scan rate (°C/min) | 10 |
| Protective gas | Nitrogen |

### Thermogravimetric Analysis (TGA)

As one of the embodiments, TGA plots in some embodiments of the present application were collected on a DISCOVERY TGA550 thermogravimetric analyzer with the test parameters shown in Table 12.

**Table 12. TGA test parameters in some embodiments of the present application.**

| Parameter term | Parameters |
|---|---|
| Method | Linear warming |
| Sample plate | Platinum Crucible |
| Scanning range | RT-400 degrees |
| Scan rate (°C/min) | 10 |
| Protective gas | Nitrogen |

### Infrared Spectroscopy (IR) Analysis

Tests were carried out using a solid potassium bromide (KBr) compression method using an Agilent Cary 630 FTIR infrared spectrometer.

It is understood that other values may be obtained when using other types of instruments that serve the same purpose as those described above or when using test conditions that differ from those used in the present application, and therefore the values quoted should not be considered absolute.

Due to the error of the instrument or the difference of the operator, the person skilled in the art can understand that the above parameters used to characterize the physical properties of the crystal may be slightly different, and thus the above parameters are only used to assist in characterizing the polymorph provided in the present application, and are not to be regarded as a limitation of the polymorph of the present application.

### Some Examples are further provided below.

The present application is further described below in connection with some Examples. It should be understood that these Examples are used only to illustrate the present application and are not intended to limit the scope of the present application. For experimental methods in the following Examples in which details of the conditions are not indicated, the guidelines given in the documents of the present application are preferably referred, and those experimental methods may also be in accordance with laboratory manuals or conventional conditions in the art, and may also be in accordance with other experimental methods known in the art or in accordance with the conditions recommended by the manufacturer.

Percentage contents covered in the present application, if not otherwise specified, refer to volume percent for gas-gas mixtures, mass percent wt% for solid-solid mixtures, volume percent % (v/v) for liquid-liquid mixtures, and mass percent wt% or solid-liquid percent % (w/v) for solid-liquid mixtures.

In the following Examples, 50% ethanol refers to 50% (v/v) ethanol/water solvent mixture.

### Reagents and Instruments

In the present application, the structure and purity of the compounds were determined by nuclear magnetic resonance (¹H NMR) and/or liquid mass spectrometry (LC-MS).¹H NMR: BrukerAVANCE-400 NMR with tetramethylsilane (TMS) as the internal standard. LC-MS: Agilent 1200 HPLC System/6140 MS liquid mass spectrometer (manufacturer: Agilent), column WatersX-Bridge, 150 mm × 4.6 mm, 3.5 µm.

The XRPD tests were performed using the parameters shown in Table 9.

The DSC test uses the parameters shown in Table 11.

The starting materials known from the present application can be synthesized using or according to methods known in the art, or can be purchased from companies such as ABCR GmbH & Co. KG, Acros Organics, Aldrich Chemical Company, Shaoyuan Chemical Technology (Accela ChemBio Inc), and Darui Chemicals.

As used herein, DCM denotes dichloromethane, DMF denotes dimethylformamide, DMSO denotes dimethylsulfoxide, THF denotes tetrahydrofuran, EA denotes ethyl acetate, PE denotes petroleum ether, Et₃N denotes triethylamine, NH₄F denotes ammonium fluoride, t-BuOH denotes tert-butyl alcohol, Pd(dppf)Cl₂ denotes [1,1'-bis(diphenylphosphine)ferrocene]palladium dichloride, CuI denotes cuprous iodide, Pd(PPh)₃₂Cl₂ denotes bis(triphenylphosphine)palladium(II) chloride, DPPA denotes diphenyl azidophosphate, and DBU denotes 1,8-diazabicycloundec-7-ene.

### As used herein, Captisol denotes sulfobutylcyclodextrin.

In the following Examples, all of hydrochloric acid solution, sulfuric acid solution and hydrobromic acid solution refer to aqueous solutions.

### Preparation of intermediate V1

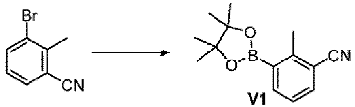

2-Methyl-3-bromobenzonitrile (15 g), bis(pinacolato)diboron (23.32 g), potassium acetate (15.02 g) and Pd(dppf)Cl₂ (2.80 g) were dissolved in a mixed solution of DMSO (20 mL) and dioxane (100 mL) under nitrogen protection and the resultant was stirred at 100°C for 3.5 h. After the reaction was complete, the solvent was evaporated under reduced pressure to give solid product, which was isolated by column chromatography (EA:PE = 15% to 40%, v/v) to give compound **V1** (21.05 g). MS (ESI): 244.1 [M+H]⁺ .

### Preparation of intermediate V2

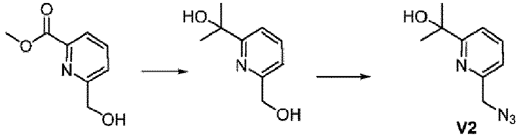

Step 1: Methyl 6-(hydroxymethyl)pyridine carboxylate (50 g) dissolved in THF (800 mL) was added dropwise to methylmagnesium bromide (3.0 M, 398.82 mL) under argon protection at a controlled temperature of 0°C to 15°C, with a large amount of gas emission, and then the reaction solution was stirred at room temperature overnight. The reaction solution was slowly added to solid ice, and then saturated ammonium chloride solution was added. The resultant was extracted with DCM (300 mL × 10), dried with anhydrous sodium sulfate, concentrated to dryness under reduced pressure, separated and purified by silica gel column chromatography (EA:PE = 0% to 100%, v/v) to give 2-(6-(hydroxymethyl)pyridin-2-yl)propan-2-ol (30 g) as a colorless oil, MS (ESI). 168.1 [M+H]⁺ .

Step 2: Compound 2-(6-(hydroxymethyl)pyridin-2-yl)propan-2-ol (30 g) was dissolved in DCM (100 mL), and DPPA (54.31 g) was added under stirring and argon protection at room temperature. Then DBU (40.17 mL) was added slowly and dropwise under the condition of an ice bath, and after the dropwise addition was completed, the reaction system was warmed up to 50°C and stirred overnight. The above reaction solution was added to 100 mL of water, washed with DCM (200 mL × 2), dried with anhydrous sodium sulfate, concentrated to dryness under reduced pressure, separated and purified by silica gel column chromatography (EA:PE = 0% to 30%, v/v) to obtain compound **V2** (30 g). MS (ESI): 193[M+H]⁺.

### Example 1. Preparation of the compound of formula X (also referred to as Compound X)

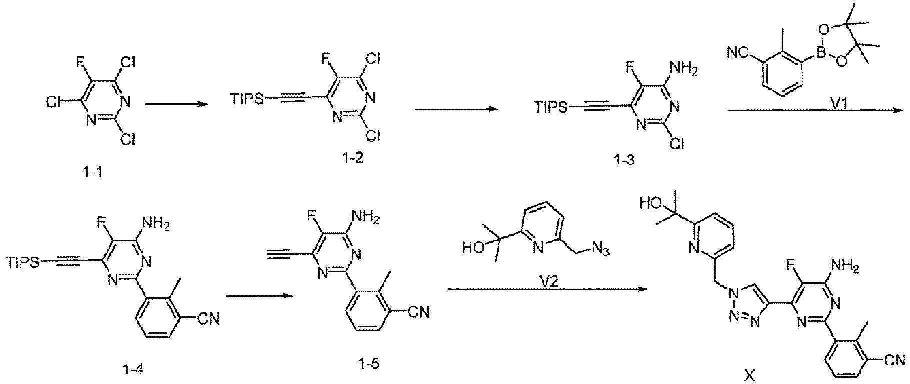

Step 1: Compound **1-1** (2,4,6-trichloro-5-fluoropyrimidine, 40 g), and triisopropylsilylacetylene (38.03 g) were dissolved in THF (400 mL) and cooled down to 0°C under argon protection, and CuI (3.78 g) and Pd(PPh )₃₂Cl₂ (6.97 g) were added sequentially, followed by the slow and dropwise addition of Et₃N (60.17 g, 595.79 mmol). After the dropwise addition, the reaction solution was stirred at 0°C for 2 h, and warmed up to room temperature and stirring continued for 6 h. Then the temperature was lowered to 0°C and NH₃ /H₂O (ammonia solution, 120 g, 80% concentration) was added dropwise with stirring at room temperature overnight. The completion of reaction was detected by LC-MS, and Compound **1-2** was obtained, MS (ESI): 347 [M+H]⁺ .

Step 2: A large amount of water was added to the reaction solution obtained at the end of step 1, and the resultant was extracted with DCM (200 mL × 3), dried with anhydrous sodium sulfate, and concentrated under reduced pressure to precipitate a large amount of solid. 60 mL of petroleum ether was added with stirring, and the resultant was filtered to obtain compound **1-3** (31 g), and the rest of the mother liquor was separated and purified by silica gel column chromatography (DCM:PE = 30% to 80%, v/v) to obtain Compound **1-3** (43 g), MS (ESI): 328.1 [M+H]⁺ .

Step 3: Compound **1-3** (41 g) and Compound **V1** (42.56 g) were dissolved in a mixed solvent of dioxane (400 mL) and water (60 mL), and Na₂CO₃ (26.51 g) and Pd(dppf)₂Cl (4.57 g) were added sequentially under argon protection with stirring at room temperature, and then the temperature was raised to 100°C with stirring for 24 h. The reaction solution was poured into ice water, extracted with DCM (300 mL × 2), dried with anhydrous sodium sulfate, concentrated to dryness under reduced pressure, separated and purified by silica gel column chromatography to give Compound **1-4** (44 g). MS (ESI): 409.2 [M+H]⁺ .

Step 4: Compound **1-4** (44 g) was added to methanol (400 mL) and NH₄F (47.60 g) was added with stirring at room temperature. The temperature was raised to 70°C under argon protection with stirring for 8 h. The reaction solution was stirred while adding a large amount of water, and a large amount of solid was washed out and filtered to give the brown Compound **1-5** (27 g). MS (ESI): 253.1 [M+H]⁺ .

Step 5: Compound **1-5** (27 g) and intermediate **V2** (22 g) were dissolved in a solvent mixture of THF (200 mL) and t-BuOH (200 mL) with stirring at room temperature under argon protection, and an aqueous solution of Cu₂SO₄·5H₂O (2.87 g) (200 mL of water), and sodium ascorbate (4.56 g) were added sequentially. The reaction solution was then heated up to 60°C and stirred for 24 h. The reaction solution was poured into water, added with saturated sodium carbonate, extracted with DCM (300 mL × 3), dried with anhydrous sodium sulfate, separated and purified by silica gel column chromatography (DCM (containing 2% triethylamine): EA = 0 to 10%, v/v) to give 30 g of Compound **X.** ¹H NMR (400 MHz, DMSO-d6) δ (ppm): 8.78 (s, 1H), 7.92 (d *J* = 7.8 Hz, 1H), 7.83 (dd, *J* = 7.7, 1.2 Hz, 1H), 7.76 (t, *J* = 7.8 Hz, 1H), 7.60 - 7.48 (m, 3H), 7.45 (t, *J* = 7.5 Hz, 1H), 7.10 (d, *J* = 6.9 Hz, 1H), 5.77 (s, 2H), 5.18 (s, 1H), 2.63 (s, 3H), 1.34 (s, 6H). MS (ESI): 445.2 [M+H]⁺. The solid Compound **X** obtained was sent for XRPD detection and the X-ray powder diffraction pattern thereof did not show characteristic peaks. Therefore, the Compound **X** obtained in this example is in amorphous form.

### Example 2: Preparation of free alkali crystal form I

### 2.1 Method I

About 20 mg of Compound **X** (amorphous) was weighed in a glass vial, to which an appropriate amount of isopropanol, ethanol, ethyl acetate or 50% ethanol was added. The system was heated to about 50°C until clarification. The glass vial was taken out, placed at room temperature to cool down to room temperature and then placed in a refrigerator to cool down to 0°C to 4°C. The solid was precipitated out and then the system was centrifuged. The supernatant was discarded. The solid was placed in an oven for drying, and an XRPD test was performed. The XRPD pattern of the resulting solid product is shown in FIG. 1, which is defined in the present application as free alkali crystal form I. The DSC plot of free alkali crystal form I is shown in FIG. 2, in which the endothermic peak is 190.36°C. The DVS plot of free alkali crystal form I is shown in FIG. 3, in which the change in mass due to absorption of the free alkali crystal form I during changes in relative humidity from 0% to 80% RH at 25°C is less than 0.2%, indicating that the free alkali crystal form I has almost no hygroscopicity.

### 2.2 Method II

About 30 g of Compound **X** (amorphous) was weighed in a glass vial, and 150 mL of acetonitrile or ethanol was added, which was heated to reflux and then cooled to room temperature to precipitate a solid and filtered to obtain the solid product. The solid obtained was subjected to XRPD test and free alkali crystal form I was obtained.

### 2.3 Method III

About 20 mg of Compound **X** (amorphous) was weighed in a glass vial, and an appropriate amount of 50% ethanol was added for dissolving to obtain a clarified solution. The solution was placed at room temperature for slow volatilization and the solid obtained after the volatilization was taken for XRPD test and free alkali crystal form I was obtained.

### 2.4 Method IV

20 mg of Compound **X** (amorphous) was weighed in a glass vial, and dimethylsulfoxide or N,N-dimethylacetamide was added for dissolving to obtain a clarified solution. The anti-solvent n-heptane was added to the solution, and solid particles precipitated. The solid obtained was subjected to XRPD test and free alkali crystal form I was obtained.

### Example 3: Preparation of free alkali crystal form IV

### 3.1 Method I

20 mg of Compound **X** (amorphous) was weighed in a glass vial, and an appropriate amount of acetone was added for dissolving to obtain a clarified solution. The solution was left to undergo slow volatilization at room temperature to obtain a solid. The XRPD pattern of the resulting solid product is shown in FIG. 4 and is defined in the present application as free alkali crystal form IV. The differential scanning calorimetry plot of free alkali crystal form IV is shown in FIG. 5, with an exothermic peak at 156.16° C and an endothermic peak at 189.64° C (the position of the endothermic peak is similar to that of free alkali crystal form I), indicating that free alkali crystal form IV is a sub-stable crystal form, which may be transformed into free alkali crystal form I during the process of warming up.

### 3.2 Method II

20 mg of Compound **X** (amorphous) was weighed in a glass vial, and an appropriate amount of ethanol or acetonitrile was added for dissolving to obtain a clarified solution. After dissolution, the anti-solvent n-heptane was added, and solid particles precipitated. The solid obtained was subjected to XRPD assay and was free alkali crystal form IV.

### Example 4: Preparation of free alkali crystal form V

About 20 mg of Compound **X** (amorphous) was weighed in a glass vial, and an appropriate amount of ethanol was added for dissolving to obtain a clarified solution. The solution was placed at room temperature for slow volatilization, and the solid obtained from the volatilization was taken and subjected to XRPD test, the XRPD pattern of which is shown in FIG. 6, which is defined as free alkali crystal form V in the present application. The DSC plot of the free alkali crystal form V is shown in FIG. 7, wherein the free alkali crystal form V has an exothermic peak at 128.28°C (it is probably because the crystals were not dried completely, and there was part of crystal water), a crystalline exothermic peak at 164.51°C, and an endothermic peak at 189.94° C (the position of the endothermic peak is similar to that of the free alkali crystal form I), which shows that the free alkali crystal form V is a sub-stable crystal form, and may be transformed into free alkali crystal form I in the process of warming up.

### Example 5: Preparation of free alkali crystal form VI

About 20 mg of Compound X (amorphous) was weighed in glass vials, respectively, and acetonitrile, tetrahydrofuran, dimethylsulfoxide, or N,N-dimethyl acetamide was added for dissolving to obtain a clarified solution. The solution was placed at room temperature for slow volatilization, and the solid obtained from the volatilization was taken and subjected to XRPD test. The XRPD pattern of the resulting solid product is shown in FIG. 8, which is defined in the present application as free alkali crystal form VI. The DSC plot of free alkali crystal form VI is shown in FIG. 9, with a crystallization exothermic peak at 159.40° C and an endothermic peak at 189.33° C (the position of the endothermic peak is similar to that of free alkali crystal form I), indicating that free alkali crystal form VI is a sub-stable crystal form, which may be transformed into free alkali crystal form I during the process of warming up.

### Example 6: Preparation of free alkali crystal form VII

In a glass vial, 20 mg of Compound **X** (amorphous) was weighed, and methanol was added for dissolving to obtain a clarified solution. N-heptane was added as an anti-solvent and solid particles precipitated. The solid obtained was subjected to XRPD test. The X-ray powder diffraction pattern of the solid product obtained is shown in FIG. 10, which is defined in the present application as free alkali crystal form VII. The differential scanning calorimetry analysis plot of the free alkali crystal form VII is shown in FIG. 11, and the free alkali crystal form VII has an endothermic peak at 189.36°C (the position of the endothermic peak is similar to that of free alkali crystal form I), which indicates that free alkali crystal form VII is a sub-stable crystal form, and it may be transformed into free alkali crystal form I during the process of warming up.

### Example 7: Mixing and Shaking

About 20 mg of Compound **X** (free alkali crystal form I) was weighed in a glass vial and 1 mL of the solvents in the table below was respectively added to multiple experimental groups. The glass vials were subjected to mixing and shaking at room temperature for 24 h. After centrifugation, the supernatant was discarded, and the solids were placed in an oven to dryness and subjected to the XRPD test. The results for each experimental group are shown in Table 13, and the free alkali crystal form I did not change after mixing and shaking in any of the seven solvents shown.

**Table 13. Polymorph screening results of mixing and shaking**

| No. | Reagents | RT, 1 day | RT, 7 days | 50°C, 1 day |
|---|---|---|---|---|
| 1 | Water | Crystal form I | Crystal form I | Crystal form I |
| 2 | n-Heptane | Crystal form I | Crystal form I | Crystal form I |
| 3 | Methyl tert-butyl ether | Crystal form I | Crystal form I | Crystal form I |
| 4 | Ethanol | Crystal form I | Crystal form I | Crystal form I |
| 5 | Isopropanol | Crystal form I | Crystal form I | Crystal form I |
| 6 | Ethyl acetate | Crystal form I | Crystal form I | Crystal form I |
| 7 | 50% Ethanol | Crystal form I | Crystal form I | Crystal form I |

### Example 8: Crystal Competition Experiment

In order to detect the stablest crystal form of Compound **X,** crystalline competition experiments were carried out. 10 mg of free alkali crystal I, free alkali crystal IV, free alkali crystal form V, free alkali crystal form VI, and free alkali crystal form VII were weighed, respectively, and mixed thoroughly. 20 mg of the mixed crystal was weighed in a glass vial, to which 1 mL of solvent in Table 14 was added respectively, and the resultant was mixed and shaken at room temperature for 24 h, and centrifugated. The precipitate was taken, dried and then measured by XRPD. The XRPD results showed that the mixed crystal forms converted into free alkali crystal I after mixing and shaking in different solvents for 24 h. Therefore, it was determined that free alkali crystal I was the stable crystal form.

**Table 14. Polymorph screening results of mixing and shaking**

| No. | Reagents | Crystal form |
|---|---|---|
| 1 | Ethanol | I |
| 2 | Isopropanol | I |
| 3 | Ethyl acetate | I |
| 4 | Water | I |
| 5 | Acetone | I |

### Example 9 Stability experiments of free alkali crystal form I

About 20 mg of free alkali crystal form I was weighed in glass sample vials, respectively, and placed under accelerated conditions (40°C, 75% RH) with the lid open. Samples were taken at 1 week, 2 weeks, 1 month, 2 months, and 4 months for XRPD test, respectively. As can be seen from FIG. 12, the free alkali crystal form I is physically stable and the XRPD patterns obtained at different sampling times are highly consistent.

### Example 10. Hydrochloride Crystal Form I

### 10.1 Method I

20 mg of Compound **X** (amorphous) was weighed and added to a 1 M hydrochloric acid solution in a molar ratio of acid to Compound **X** of 1.2:1, followed by the addition of 2 mL of acetone or isopropanol. The solution was heated and sonicated for dissolving to obtain a clarified solution. The solution was held at 50°C for 4 h, after that the temperature was slowly lowered to allow for the precipitation of a solid, which was collected by centrifugation. The resulting solid was used for XRPD test after evaporating the solvent, and the X-ray powder diffraction pattern is shown in FIG. 13, which is defined in the present application as hydrochloride crystal form I. The differential scanning calorimetry (DSC) analysis of hydrochloride crystal form I is shown in FIG. 14, which shows an endothermic peak at 225.37°C.

### 10.2 Method II

20 mg of Compound **X** (amorphous) was weighed, and 1 M hydrochloric acid solution was added in a molar ratio of acid to Compound **X** of 1.2:1, followed by the addition of 2 mL of acetone or isopropanol. The solution was heated and sonicated for dissolving to obtain a clarified solution. The solution was held at 50°C for 4 h and cooled to room temperature. The anti-solvent n-heptane was added to allow the precipitation of solid, which was centrifuged. The solid was collected, and the solvent in the resulting solid was evaporated to obtain the hydrochloric acid crystal form I.

### Example 11. Sulfate Crystal Form I

### 11.1 Method I

20 mg of Compound **X** was weighed and 0.5 M sulfuric acid solution was added in a molar ratio of acid to Compound **X** of 0.6:1, followed by the addition of 2 mL of isopropanol, ethanol, or acetone. The system was heated and sonicated for dissolving to obtain a clarified solution. The solution was held at 50°C for 4 h, after which the temperature was slowly lowered to allow precipitation of a solid. The solid was collected by centrifugation. After volatilization of the solvent to dryness, the resulting solid was used for XRPD test and the X-ray powder diffraction pattern thereof is shown in FIG. 15, which is defined in the present application as sulfate crystal form I. The differential scanning calorimetry plot of sulfate crystal form I is shown in FIG. 16, with an endothermic peak at 205.20°C for sulfate crystal form I.

### 11.2 Method II

20 mg of Compound **X** was weighed and 0.5 M sulfuric acid solution was added in a molar ratio of acid to Compound **X** of 0.6:1, followed by the addition of 2 mL of ethyl acetate. The system was heated and sonicated for dissolving to obtain a clarified solution. The solution was held at 50°C for 4 h. The clarified solution was cooled down to room temperature, and the anti-solvent n-heptane was added to obtain the sulfate crystal form I.

### 11.3 Method III

300 mg of Compound **X** (amorphous) was weighed and added to a small beaker, and 5 mL of acetone was added. The mixture was sonicated for dissolving. At 50°C, while stirring, 900 mL of 0.5 M sulfuric acid solution was added slowly and dropwise, and the system was held for 4 h. The solution was in turbid state, which was centrifuged and the supernatant was discarded. The solid was separated, washed with acetone for 3 to 5 times, and dried under vacuum at 40°C overnight to obtain the sulfate crystal form I.

### Example 12: Hydrobromide Crystal Form I

20 mg of Compound **X** was weighed and 1 mol/L hydrobromic acid was added in a molar ratio of acid to Compound **X** of 1.2:1, followed by the addition of 1 mL to 3 mL of isopropanol, ethanol, acetone, or ethyl acetate. The system was heated and sonicated for dissolving to obtain a clarified solution. The solution was held at 50°C for 4 h. The system was then cooled down slowly to precipitate a solid, and centrifuged to collect the solid. The resulting solid was used for XRPD test after volatilization of the solvent. The X-ray powder diffraction pattern is shown in FIG. 17 and is defined as hydrobromide crystal form I in the present application.

### Example 13. Stability experiments of hydrochloride crystal form I and sulfate crystal form I

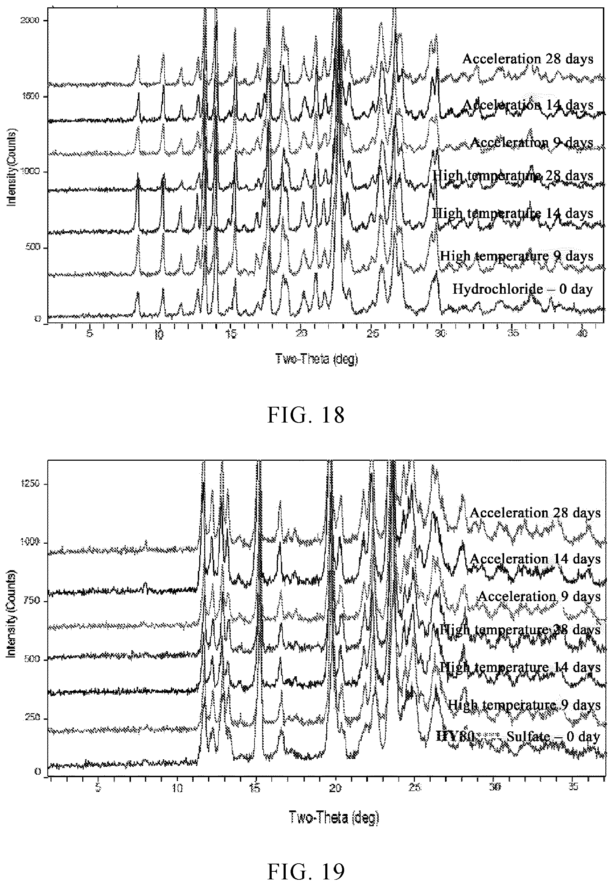

Appropriate amounts of hydrochloride crystal form I and sulfate crystal form I samples were weighed and placed under accelerated (40°C/75% RH) condition with open lid and in high temperature (60°C) and airtight condition. XRPD test was performed at day 9, day 14 and day 28, respectively. The results are shown in FIG. 18 and FIG. 19. As can be seen from FIG. 18 and FIG. 19, the hydrochloride crystal form I and sulfate crystal form I have good crystalline stability, and the XRPD patterns obtained at different sampling times are highly consistent in both FIG. 18 and FIG. 19.

### Biological Test

In the following test examples, the sample used was Compound **X,** and Compound **D1** was used as a control.

### Test Example 1: Inhibitory Activity on A2A and A2B Receptors

CHO-K1/ADORA2A/Gα15 (GenScript, M00246) and CHO-K1/ADORA2B/Gα15 (GenScript, M00329) cells were cultured in Ham's F-12 (Gibco, 31765092) medium with medium conditions containing 10% FBS, 200 µg/mL Zeocin (bleomycin) and 100 µg/mL Hygromycin B (thaumatin B) or 10% FBS, 400 µg/mL G418 and 100 µg/mL Hygromycin B. Details of the culture conditions are described in the corresponding instructions. The screening procedure was as follows:
1. adjusting the cell density to 6 × 10⁵ cells/mL with serum-free medium;
2. adding 5 mL of cytosol, 2.5 mL of NECA (Sigma, 119140-10MG), and 2.5 mL of compound solution sequentially to each well of a 384-well plate (Greiner Bio-One, 784075), in which the final concentration of NECA is 50 nM (CHO-K1/ADORA2A) or 10 nM (CHO-K1/ADORA2B), and the final concentrations of the compound starts from 3 µM to triple dilutions less than 3 µM;
3. placing the 384-well plate in 37°C incubator for 30 min;
4. adding 5 mL of Camp-d₂ and 5 mL of Camp-ab (Cisbio, 62AM4PEB) sequentially;
5. leaving the 384-well plate at room temperature for 1 h, protected from light; and
6. reading the plate (Victor X5, PerkinElmer) and analyzing the data by Xlfit nonlinear regression to calculate the IC₅₀ of the compound. The results are shown in Table 15.

**Table 15. Inhibitory activity of the compound on A_{2A} receptor and A_{2B} receptor.**

| Compound No. | A_{2A} receptor (IC₅₀/mM) | A_{2B} receptor (IC₅₀/mM) |
|---|---|---|
| X | 0.001 | 0.003 |

As can be seen from Table 14, Compound X of the present application has high inhibitory activity on both A_{2A} receptor and A_{2B} receptor.

### Test Example 2: Inhibitory Activity on A2A and A2B Receptors

CHO-K1/ADORA2A/Gα15 (GenScript, M00246) and CHO-K1/ADORA2B/Gα15 (GenScript, M00329) cells were cultured in Ham's F-12 (Gibco, 31765092) medium with medium conditions containing 10% FBS, 200 µg/mL Zeocin and 100 µg/mL Hygromycin B or 10% FBS, 400 µg/mL G418 and 100 µg/mL Hygromycin B. Details of the culture conditions are described in the corresponding manuals. The screening procedure was as follows:
1. adjusting the cell density to 6 × 10⁵ cells/mL with serum-free medium;
2. adding 5 mL of cytosol, 2.5 mL of NECA (Sigma, 119140-10MG), and 2.5 mL of compound solution sequentially to each well of a 384-well plate (Greiner Bio-One, 784075), in which the final concentration of NECA is 1 µM (CHO-K1/ADORA2A) or 0.1 µM (CHO-K1/ADORA2B), and the final concentration of the compound starts from 3 µM to triple dilutions less than 3 µM;
3. placing the 384-well plate in 37°C incubator for 30 min;
4. adding 5 µL of cAMP-d₂ and 5 µL of cAMP-ab (Cisbio, 62AM4PEB) sequentially;
5. leaving the 384-well plate at room temperature for 1 h, protected from light; and
6. reading the plate (Victor X5, PerkinElmer) and analyzing the data by XLfit nonlinear regression to calculate the IC₅₀ of the compound. The results are shown in Table 16.

**Table 16. Inhibitory activity of compound on A_{2A} receptor and A_{2B} receptor**

| Compound No. | A_{2A} receptor (IC₅₀/µM) | A_{2B} receptor (IC₅₀/µM) |
|---|---|---|
| X | 0.024 | 0.062 |
| D1 | 0.065 | 0.075 |

The structure of compound D1 is shown below, CAS No. 2239273-34-6; Supplier: Suzhou Chukai Pharmaceutical Co., Ltd; Batch No.: 2009010AHP07.

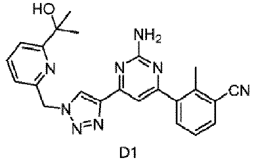

### Test Example 3: In vivo pharmacokinetics

The LC/MS/MS method was applied to determine the medicament concentration in plasma at different moments after intravenous administration and oral gavage administration of Compound X of the present application in mice, respectively, to study the pharmacokinetic behavior of Compound X of the present application in mice, and to evaluate its pharmacokinetic characteristics.

### Experimental scheme:

Test animals: healthy adult male ICR mice (weighing from 25 g to 40 g, 12 mice, free to drink and eat in the intravenous injection group, fasted overnight in the gavage group, and free to drink and eat 4 h after the administration of the medicament), supplied by Beijing Viton Lever Laboratory Animal Technology Co., Ltd.

Mode of administration and dosage: animals meeting the experimental requirements were selected and weighed and labeled before administration. ICR mice were administered by tail vein (2 mg/kg, 5% DMSO, pH 4.5 20% Captisol) and by gavage (10 mg/kg, 5% DMSO, pH 4.5 20% Captisol).

Blood sample collection: Prior to blood sample collection, mice were bound and approximately 100 mL of blood was collected from the jugular vein of each administered mouse at predetermined blood collection time points (i.v. administration: Blood was collected at 9 time points: 0.083 h, 0.25 h, 0.5 h, 1 h, 2 h, 4 h, 6 h, 7.5 h, and 24 h, respectively, after administration; and gavage administration: Blood was collected at 9 time points: 0.083 h, 0.25 h, 0.5 h, 1 h, 2 h, 4 h, 6 h, 7.5 h, and 24 h, respectively, after administration). The blood was transferred to 1.5 mL tubes preloaded with K2EDTA, and the blood plasma was separated by centrifugation for 4 min (8,000 rpm, 4 °C), which was performed within 15 min after the collection. All samples were stored in a refrigerator at -20°C until sample analysis. LC/MS/MS method was applied to determine the medicament concentration.

The characteristic pharmacokinetic parameters of Compound X of the present application in mice under intravenous administration are shown in Table 17.

**Table 17: Pharmacokinetic parameters for intravenous administration.**

| Compound No. | Clearance CLz (mL/min/kg) | Area under the curve AUC₀₋ₜ (hr·ng/mL) |
|---|---|---|
| Compound X | 14.7 | 2252 |
| D1 | 19.0 | 1646 |

The characteristic pharmacokinetic parameters of Compound X of the present application in mice under gavage administration are shown in Table 18.

**Table 18. Pharmacokinetic parameters under gavage administration.**

| Compound No. | Cₘₐₓ (ng/mL) | Area under the curve AUClast (hr·ng/mL) |
|---|---|---|
| Compound X | 8730 | 10446 |
| D1 | 5957 | 10291 |

### Test Example 4: In vivo potency assay of Compound X of the present application

Experimental Protocol: In this test example, tumor bearing mice subcutaneously transplanted with mouse colon cancer MC38(#22)-hpd-L1 was investigated. After administration of Compound X of the present application via the oral administration, the *in vivo* efficacy of Compound X on colon cancer MC38(#22)-hpd-L1 tumor bearing mice were tested.

Experimental materials: C57BL/6 mice (female); mouse colon cancer MC38(#22)-hpd-L1 cells (Shanghai Jiao Tong University cell bank), cultured *in vitro* in monolayer, culture conditions were using RPMI-1640 medium containing 10% fetal bovine serum, 37°C 5% CO₂ incubator. The cells were passaged by conventional digestion treatment with trypsin-EDTA. When the cells were in the exponential growth phase with 80% to 90% saturation, the cells were harvested and counted.

Compound Preparation: The compound was weighed and added to the solvent (40% captisol in acetate buffer, pH 3.8) to prepare a 10 mg/mL sample. 75 µL of Tecentriq solution (60 mg/mL) was taken and added to 8.925 mL of phosphate buffer solution (PBS) to make a 0.5 mg/mL phosphate solution (phosphate buffer).

Experimental Operation: Cells were resuspended in the phosphate buffer at a density of 5×10⁶ cells/mL. 0.1 mL of PBS (containing 5×10⁵ MC38(#22)-hpd-L1 cells) was subcutaneously inoculated into the right back of each mouse. On the day of inoculation, the mice were randomized according to their body weights into groups of 10 mice each, and dosing was begun twice a day for 22 days. The animals were weighed daily and monitored for health throughout the experimental period. Tumor diameters were measured twice a week using vernier calipers.

Tumor volume *V* was calculated as *V* = 0.5 × *a* × *b*² , with *a* and *b* denoting the long and short diameters of the tumor, respectively.

The tumor suppression efficacy of the compound was evaluated by relative tumor proliferation rate T/C (%). Relative tumor proliferation rate T/C (%) = Vt/Vc × 100% (Vt: mean tumor volume of the treatment group; Vc: mean tumor volume of the negative control group). Vt and Vc were data taken from the same day. The results are shown in Table 19.

**Table 19: Relative tumor proliferation rates obtained in Test Example 4**

| Compound No. | Dose (mg/kg) | Tumor volume (mm³) | T/C (%) |
|---|---|---|---|
| Solvent group | / | 1191 | / |
| Compound X | 10 | 899 | 75.5 |
| | 30 | 405 | 34.0 |
| | 100 | 286 | 24.0 |
| Compound D1 | 100 | 902 | 75.8 |

All documents referred to in the present application are cited as references in the present application as if each document were individually cited as a reference. Unless in conflict with the purpose of the invention or the technical solution of the present application, the cited documents involved in the present application are cited in their entirety and for their entire purpose. When the present application involves cited documents, the definitions of relevant technical features, terms, nouns, phrases, and the like in the cited documents are also cited. When the present application involves the cited documents, the cited examples and preferred ways of the relevant technical features may also be incorporated into the present application as references, but only to the extent that the present application can be implemented. It should be understood that when the cited contents conflict with the description in the present application, the present application shall prevail or adaptive amendments shall be made according to the description in the present application.

The various technical features of the above embodiments and examples may be combined in any suitable manner. For the sake of clarity of description, not all possible combinations of the various technical features of the above embodiments and examples have been described. However, as long as the combinations of these technical features are not contradictory, they should be considered to be within the scope of the present specification as recorded herein.

The above examples only represent several embodiments of the present application to facilitate a detailed understanding of the technical solutions of the present application, but they are not to be construed as limitations on the protection scope of the present application. It should be noted that, for a person of ordinary skill in the art, several deformations and improvements can be made without departing from the conception of the present application, all of which fall within the protection scope of the present application. It should also be understood that after reading the above teachings of the present application, a person skilled in the art may make various changes or modifications to the present application, and the equivalent forms obtained will also fall within the protection scope of the present application. It should also be understood that the technical solutions obtained by the person skilled in the art through logical analysis, reasoning or limited experimentation on the basis of the technical solutions provided in the present application are within the protection scope of the claims appended to the present application. Therefore, the protection scope of the patent application shall be subject to the contents of the appended claims, and the specification and the accompanying drawings may be used to interpret the contents of the claims.

## Claims

1. A polymorph of a compound of formula X or a pharmaceutically acceptable salt thereof, 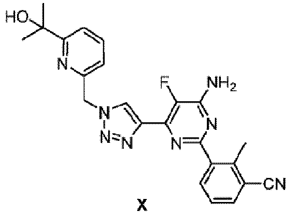 wherein the pharmaceutically acceptable salt is an inorganic salt.

2. The polymorph of claim 1, wherein the pharmaceutically acceptable salt is any one selected from: hydrochloride, sulfate and hydrobromide.

3. The polymorph of any one of claims 1 to 2, wherein the polymorph of the compound of formula X is any one selected from:
free alkali crystal form I of the compound of formula X with an X-ray powder diffraction pattern having peaks at diffraction angle 2θ (°) of the group comprising: 18.08 ± 0.2, 21.41 ± 0.2 and 24.83 ± 0.2;
free alkali crystal form IV of the compound of formula X with an X-ray powder diffraction pattern having peaks at diffraction angle 2θ (°) of the group comprising: 13.04 ± 0.2, 15.80 ± 0.2, 16.46 ± 0.2 and 23.89 ± 0.2;
free alkali crystal form V of the compound of formula X with an X-ray powder diffraction pattern having peaks at diffraction angle 2θ (°) of the group comprising: 6.17±0.2, 9.37±0.2, 10.39±0.2, 11.65±0.2, 14.35±0.2, 15.74±0.2 and 17.21±0.2;
free alkali crystal form VI of the compound of formula X with an X-ray powder diffraction pattern having peaks at diffraction angle 2θ (°) of the group comprising: 12.55±0.2, 14.86±0.2, 16.15±0.2, 17.69±0.2, 21.08±0.2, 21.58±0.2, 24.53±0.2 and 25.01±0.2; and
free alkali crystal form VII of the compound of formula X with an X-ray powder diffraction pattern having peaks at diffraction angle 2θ (°) of the group comprising: 14.92±0.2, 16.13±0.2, 17.59±0.2, 20.87±0.2, 21.20±0.2, 21.71±0.2, 24.12±0.2, 24.62±0.2 and 25.12±0.2.

4. The polymorph of claim 3, wherein the polymorph of the compound of formula X is any one selected from:
the free alkali crystal form I with the X-ray powder diffraction pattern further comprising 2 or more peaks at diffraction angle 2θ (°) selected from: 12.90±0.2, 15.26±0.2, 16.47±0.2, 17.81±0.2, 19.57±0.2, 22.01±0.2 and 25.43±0.2;
the free alkali crystal form IV with the X-ray powder diffraction pattern further comprising 2 or more peaks at diffraction angle 2θ (°) selected from: 6.32±0.2, 9.08±0.2, 9.58±0.2, 14.12±0.2, 20.14±0.2, 20.59±0.2 and 27.53±0.2;
the free alkali crystal form V with the X-ray powder diffraction pattern further comprising 2 or more peaks at diffraction angle 2θ (°) selected from: 21.65±0.2, 22.31±0.2, 24.55±0.2, 24.86±0.2, 25.70±0.2, 26.08±0.2 and 27.31±0.2;
the free alkali crystal form VI with the X-ray powder diffraction pattern further comprising 2 or more peaks at diffraction angle 2θ (°) selected from: 9.04±0.2, 9.68±0.2, 13.37±0.2, 18.53±0.2, 19.19±0.2, 19.64±0.2, 19.97±0.2, 23.69±0.2, 27.55± 0.2, 30.34±0.2 and 31.46±0.2; and
the free alkali crystal form VII with the X-ray powder diffraction pattern further comprising 2 or more peaks at diffraction angle 2θ (°) selected from: 6.17±0.2, 9.07±0.2, 9.67±0.2, 10.37±0.2, 12.61±0.2, 14.39±0.2, 19.21±0.2, 19.73±0.2 and 20.05± 0.2.

5. The polymorph of claim 4, wherein the polymorph of the compound of formula X is any one selected from:
the free alkali crystal form I with the X-ray powder diffraction pattern further comprising 2 or more peaks at diffraction angle 2θ (°) selected from: 9.50±0.2, 10.13±0.2, 12.53±0.2, 18.89±0.2, 19.94±0.2 and 20.33±0.2;
the free alkali crystal form IV with the X-ray powder diffraction pattern further comprising 1 or 2 peaks at diffraction angle 2θ (°) selected from: 7.64 ± 0.2 and 8.34 ± 0.2;
the free alkali crystal form V with the X-ray powder diffraction pattern further comprising 2 or more peaks at diffraction angle 2θ (°) selected from: 6.91±0.2, 8.08±0.2, 8.70±0.2, 12.77±0.2 and 13.25±0.2;
the free alkali crystal form VI with the X-ray powder diffraction pattern further comprising 1 or 2 peaks at diffraction angle 2θ (°) selected from: 7.24 ± 0.2 and 12.07 ± 0.2; and
the free alkali crystal form VII with the X-ray powder diffraction pattern further comprising 2 or more peaks at diffraction angle 2θ (°) selected from: 27.25±0.2, 27.39±0.2, 27.76±0.2, 28.97±0.2, 30.36±0.2, 31.25±0.2 and 31.67±0.2.

6. The polymorph of any one of claims 1 to 5, wherein,
the X-ray powder diffraction pattern of the free alkali crystal form I is substantially as **characterized in** FIG. 1;
the X-ray powder diffraction pattern of the free alkali crystal form IV is substantially as **characterized in** FIG. 4;
the X-ray powder diffraction pattern of the free alkali crystal form V is substantially as **characterized in** FIG. 6;
the X-ray powder diffraction pattern of the free alkali crystal form VI is substantially as **characterized in** FIG. 8; and
the X-ray powder diffraction pattern of the free alkali crystal form VII is substantially as **characterized in** FIG. 10.

7. The polymorph of any one of claims 1 to 6, wherein each X-ray powder diffraction pattern is obtained using Cu-Kα radiation.

8. The polymorph of any one of claims 1 to 7, wherein,
a differential scanning calorimetry curve of the free alkali crystal form I has an endothermic peak at 190. 15±3°C;
a differential scanning calorimetry curve of the free alkali crystal form IV has an endothermic peak at 189.64±3°C;
a differential scanning calorimetry curve of the free alkali crystal form V has an endothermic peak at 189.94±3°C;
a differential scanning calorimetry curve of the free alkali crystal form VI has an endothermic peak at 189.33±3°C; and
a differential scanning calorimetry curve of the free alkali crystal form VII has an endothermic peak at 189.36±3°C.

9. The polymorph of any one of claims 1 to 8, wherein the free alkali crystal form I has one or more characteristics selected from:
(1) having a TGA-DSC plot substantially as **characterized in** FIG. 2;
(2) having a DVS plot substantially as **characterized in** FIG. 3; and
(3) having an infrared spectrum substantially as **characterized in** FIG. 20.

10. The polymorph of any one of claims 1 to 2, wherein the polymorph of the pharmaceutically acceptable salt of the compound of formula X is any one selected from:
hydrochloride crystal form I of the compound of formula X with an X-ray powder diffraction pattern having peaks at diffraction angle 2θ (°) of the group comprising: 13.12±0.2, 13.91±0.2, 17.62±0.2, 22.58±0.2 and 26.51±0.2;
sulfate crystal form I of the compound of formula X with an X-ray powder diffraction pattern having peaks at diffraction angle 2θ (°) of the group comprising: 15.13 ± 0.2, 19.64 ± 0.2 and 23.48 ± 0.2; and
hydrobromide crystal form I of the compound of formula X with an X-ray powder diffraction pattern having peaks at diffraction angle 2θ (°) of the group comprising: 16.70 ± 0.2, 23.51 ± 0.2 and 23.96 ± 0.2.

11. The polymorph of claim 10, wherein the polymorph of the pharmaceutically acceptable salt of the compound of formula X is any one selected from:
the hydrochloride crystal form I with the X-ray powder diffraction pattern further comprising 2 or more peaks at diffraction angle 2θ (°) selected from: 8.39±0.2, 10.18±0.2, 15.25±0.2, 18.64±0.2, 20.96±0.2, 25.52±0.2, 27.01±0.2 and 29.48±0.2;
the sulfate crystal form I with the X-ray powder diffraction pattern further comprising 2 or more peaks at diffraction angle 2θ (°) selected from: 11.62±0.2, 12.77±0.2, 13.13±0.2, 22.25±0.2, 24.80±0.2 and 26.09±0.2; and
the hydrobromide crystal form I with the X-ray powder diffraction pattern further comprising 2 or more peaks at diffraction angle 2θ (°) selected from: 11.84±0.2, 12.79±0.2, 19.34±0.2, 20.23±0.2, 23.09±0.2, 24.34±0.2, 25.37±0.2, 26.21±0.2, 26.99±0.2, 28.04±0.2, 33.22±0.2 and 35.96±0.2.

12. The polymorph of claim 11, wherein the polymorph of the pharmaceutically acceptable salt of the compound of formula X is any one selected from:
the hydrochloride crystal form I with the X-ray powder diffraction pattern further comprising 2 or more peaks at diffraction angle 2θ (°) selected from: 11.44±0.2, 12.63±0.2, 17.27±0.2, 18.97±0.2, 20.12±0.2, 21.61±0.2, 23.29±0.2 and 29.15±0.2;
the sulfate crystal form I with the X-ray powder diffraction pattern further comprising 2 or more peaks at diffraction angle 2θ (°) selected from: 12.19±0.2, 16.45±0.2 and 21.71±0.2; and
the hydrobromide crystal form I with the X-ray powder diffraction pattern further comprising 2 or more peaks at diffraction angle 2θ (°) selected from: 11.48±0.2, 13.64±0.2, 15.46±0.2, 15.96±0.2, 17.66±0.2, 18.71±0.2, 20.99±0.2, 21.51±0.2, 31.60±0.2, 31.90±0.2, 35.52±0.2, 36.98±0.2, 37.81±0.2, 39.29±0.2 and 39.73±0.2.

13. The polymorph of any one of claims 10 to 12, wherein,
the X-ray powder diffraction pattern of the hydrochloride crystal form I is substantially as **characterized in** FIG. 13;
the X-ray powder diffraction pattern of the sulfate crystal form I is substantially as **characterized in** FIG. 15; and
the X-ray powder diffraction pattern of the hydrobromide crystal form I is substantially as **characterized in** FIG. 17.

14. The polymorph of any one of claims 10 to 13, wherein each X-ray powder diffraction pattern is obtained using Cu-Kα radiation.

15. The polymorph of any one of claims 10 to 14, wherein,
a differential scanning calorimetry curve of the hydrochloride crystal form I has an endothermic peak at 225.37±3°C; and
a differential scanning calorimetry curve of the sulfate crystal form I has an endothermic peak at 205.20±3°C.

16. A preparation method for a polymorph of a compound of formula X, 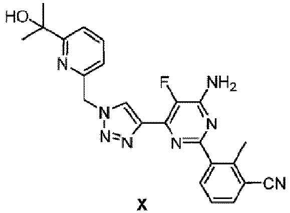 wherein the preparation method comprises the following steps:
dissolving the compound of formula X in the presence of a solvent to form a clarified solution; and
crystallizing the solution to prepare the polymorph of the compound of formula X.

17. The preparation method for the polymorph of claim 16, wherein the polymorph of the compound of formula X is free alkali crystal form I.

18. The preparation method for the polymorph of claim 16 or 17, wherein the solvent is selected from acetonitrile, water, methanol, ethanol, isopropanol, acetone, ethyl acetate, 50% (v/v) ethanol/water solvent mixture, dimethylsulfoxide, N,N-dimethylacetamide, tetrahydrofuran, and a mixture thereof.

19. The preparation method for the polymorph of any one of claims 16 to 18, wherein:
(Ia) the solvent is acetonitrile, isopropanol, ethanol, ethyl acetate or a 50% (v/v) ethanol/water mixture, and the crystallizing is performed by cooling crystallization; or
(Ib) the solvent is a 50% (v/v) ethanol/water solvent mixture, and the crystallizing is performed by volatilizing crystallization; or
(Ic) the solvent is dimethylsulfoxide or N,N-dimethylacetamide, and the crystallizing is performed by anti-solvent crystallization.

20. The preparation method for the polymorph of any one of claims 16 to 19, wherein the method comprises the steps of:
dissolving the compound of formula X in isopropanol, ethanol, ethyl acetate or 50% (v/v) ethanol/water solvent mixture at 50±5°C to form the clarified solution; and
cooling the solution to 0°C to 4°C to allow crystals to precipitate.

21. The preparation method for the polymorph of any one of claims 16 to 19, wherein the method comprises the steps of:
dissolving the compound of formula X in acetonitrile at 75±5°C to form the clarified solution; or dissolving the compound of formula X in ethanol at 70±5°C to form the clarified solution; and
cooling the solution to room temperature to allow crystals to precipitate.

22. A pharmaceutical composition, wherein the pharmaceutical composition comprises:
(a) the polymorph of any one of claims 1 to 15, or the polymorph obtained by the preparation method of any one of claims 16 to 21; and
(b) a pharmaceutically acceptable carrier.

23. Use of the polymorph of any one of claims 1 to 15, or the polymorph obtained by the preparation method of any one of claims 16 to 21, or the pharmaceutical composition of claim 22 in the manufacture of a medicament for prevention, treatment, or prophylaxis of a tumor or an immune-related disease mediated by adenosine A_{2A} receptor, mediated by adenosine A_{2B} receptor, or co-mediated by adenosine A_{2A} receptor in conjunction with adenosine A_{2B} receptor.

24. The use of claim 23, wherein the compound of formula X acts as an A_{2A}/A_{2B} receptor antagonist.
